(19) Europäisches Patentamt
European Patent Office
Office européen des brevets

(11) EP 2 700 697 A1

(12) **EUROPEAN PATENT APPLICATION**
published in accordance with Art. 153(4) EPC

(43) Date of publication:
**26.02.2014 Bulletin 2014/09**

(21) Application number: **12774798.8**

(22) Date of filing: **04.04.2012**

(51) Int Cl.:
***C09K 19/42*** *(2006.01)* ***C07C 22/00*** *(2006.01)*
***C09K 19/12*** *(2006.01)* ***C09K 19/30*** *(2006.01)*
***C09K 19/34*** *(2006.01)* ***G02F 1/13*** *(2006.01)*

(86) International application number:
**PCT/JP2012/059186**

(87) International publication number:
**WO 2012/144321 (26.10.2012 Gazette 2012/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.04.2011 JP 2011092212**

(71) Applicants:
- **JNC Corporation**
  **Chiyoda-ku**
  **Tokyo 100-8105 (JP)**
- **JNC Petrochemical Corporation**
  **Tokyo 100-0004 (JP)**

(72) Inventors:
- **GOTO Yasuyuki**
  **Tokyo 100-8105 (JP)**
- **KAWASAKI Subaru**
  **Ichihara-shi**
  **Chiba 290-8551 (JP)**
- **HATTORI Norikatsu**
  **Ichihara-shi**
  **Chiba 290-8551 (JP)**

(74) Representative: **Thurston, Joanna**
**Withers & Rogers LLP**
**4 More London Riverside**
**London SE1 2AU (GB)**

(54) **LIQUID CRYSTAL COMPOSITION AND LIQUID CRYSTAL DISPLAY ELEMENT**

(57) The invention provides a liquid crystal composition that satisfies at least one of characteristics such as a high maximum temperature of a nematic phase, a low minimum temperature of a nematic phase, a small viscosity, a suitable optical anisotropy, a large negative dielectric anisotropy, a large specific resistance, a high stability to ultraviolet light and a high stability to heat, or that is suitably balanced between at least two of the characteristics. The invention provides an AM device that has a short response time, a large voltage holding ratio, a large contrast ratio, a long service life and so forth. Herein, the liquid crystal composition has negative dielectric anisotropy and includes a specific two-ring compound having a low minimum temperature as a first component and a specific compound having a large negative dielectric anisotropy as a second component, and may include a specific compound having a small viscosity as a third component and a specific compound having a large negative dielectric anisotropy as a fourth component, and the liquid crystal display device contains this composition.

EP 2 700 697 A1

## Description

### Technical Field

The invention relates mainly to a liquid crystal composition suitable for use in an active matrix (AM) device and so forth, and an AM device and so forth that contain this composition. More specifically, it relates to a liquid crystal composition having negative dielectric anisotropy, and a device containing this composition and having an in-plane switching (IPS) mode, a vertical alignment (VA) mode, a fringe field switching (FFS) mode or a polymer sustained alignment (PSA) mode.

### Technical Background

In a liquid crystal display device, a classification based on an operating mode for liquid crystals includes modes of phase change (PC), twisted nematic (TN), super twisted nematic (STN), electrically controlled birefringence (ECB), optically compensated bend (OCB), in-plane switching (IPS), vertical alignment (VA), fringe field switching (FFS) and polymer sustained alignment (PSA). A classification based on a driving mode in the device includes a passive matrix (PM) and an active matrix (AM). The PM is classified into static, multiplex and so forth, and the AM is classified into a thin film transistor (TFT), a metal-insulator-metal (MIM) and so forth. The TFT is further classified into amorphous silicon and polycrystal silicon. The latter is classified into a high temperature type and a low temperature type according to the production process. A classification based on a light source includes a reflection type utilizing natural light, a transmission type utilizing a backlight and a semi-transmission type utilizing both natural light and a backlight.

These devices contain a liquid crystal composition having suitable characteristics. This liquid crystal composition has a nematic phase. General characteristics of the composition should be improved to give an AM device having good general characteristics. Table 1 below summarizes the relationship between these two general characteristics. The general characteristics of the composition will be further explained on the basis of a commercially available AM device. The temperature range of a nematic phase relates to the temperature range in which the device can be used. A desirable maximum temperature of the nematic phase is approximately 70 °C or higher and a desirable minimum temperature of the nematic phase is approximately -20 °C or lower. The viscosity of the composition relates to the response time of the device. A short response time is desirable for displaying moving images on the device. Accordingly, a small viscosity of the composition is desirable. A small viscosity at a low temperature is more desirable.

**Table 1. General Characteristics of Composition and AM Device**

| No. | General Characteristics of Composition | General Characteristics of AM Device |
|---|---|---|
| 1 | wide temperature range of a nematic phase | wide usable temperature range |
| 2 | small viscosity 1) | short response time |
| 3 | suitable optical anisotropy | large contrast ratio |
| 4 | large positive or negative dielectric anisotropy | low threshold voltage and small electric power consumption large contrast ratio |
| 5 | large specific resistance | large voltage holding ratio and large contrast ratio |
| 6 | high stability to ultraviolet light and heat | long service life |
| **1) A liquid crystal composition can be injected into a liquid crystal cell in a shorter period of time.** | | |

The optical anisotropy of the composition relates to the contrast ratio of the device. The product ($\Delta n \times d$) of the optical anisotropy ($\Delta n$) of the composition and the cell gap (d) of the device is designed so as to maximize the contrast ratio. A suitable value of the product depends on the kind of operating mode. In a device having a VA mode, a suitable value is in the range of approximately 0.30 $\mu$m to approximately 0.40 $\mu$m, and in a device having an IPS mode or an FFS mode, a suitable value is in the range of approximately 0.20 $\mu$m to approximately 0.30 $\mu$m. In this case, a composition having a large optical anisotropy is desirable for a device having a small cell gap. A large absolute value of the dielectric anisotropy in the composition contributes to a low threshold voltage, small electric power consumption and a high contrast ratio of the device. Accordingly, a large absolute value of the dielectric anisotropy is desirable. A large specific resistance of the composition contributes to a large voltage holding ratio and a large contrast ratio of the device. Accordingly, it is desirable that a composition should have a large specific resistance at room temperature and also at a high temperature in the initial stage. It is desirable that a composition should have a large specific resistance at room temperature and also at a high temperature after it has been used for a long time. The stability of the composition to ultraviolet light and

heat relates to the service life of the liquid crystal display device. In the case where the stability is high, the device has a long service life. Such characteristics are desirable for an AM device used in a liquid crystal projector, a liquid crystal television and so forth.

A composition having positive dielectric anisotropy is used for an AM device having a TN mode. In contrast, a composition having negative dielectric anisotropy is used for an AM device having a VA mode. A composition having positive or negative dielectric anisotropy is used for an AM device having an IPS mode or an FFS mode. A composition having positive or negative dielectric anisotropy is used for an AM device having a PSA mode. Examples of the liquid crystal composition are disclosed in the following patent document No. 1 and patent document No. 2.

## Prior Art

### Patent Document

Patent document Neo. 1: JP H11-035500 A (1999).
Patent document No. 2: JP H03-505742 A (1991).

A desirable AM device has characteristics such as a wide temperature range in which the device can be used, a short response time, a large contrast ratio, a low threshold voltage, a large voltage holding ratio and a long service life. Response time that is even one millisecond shorter than that of the other devices is desirable. Thus, desirable characteristics of the composition include a high maximum temperature of a nematic phase, a low minimum temperature of a nematic phase, a small viscosity, a suitable optical anisotropy, a large positive or large negative dielectric anisotropy, a large specific resistance, a high stability to ultraviolet light and a high stability to heat.

## Disclosure of the Invention

### Subject to be solved by the Invention

One of the aims of the invention is to provide a liquid crystal composition that satisfies at least one of characteristics such as a high maximum temperature of a nematic phase, a low minimum temperature of a nematic phase, a small viscosity, a suitable optical anisotropy, a large negative dielectric anisotropy, a large specific resistance, a high stability to ultraviolet light and a high stability to heat. Another aim is to provide a liquid crystal composition that is suitably balanced between at least two of the characteristics and a liquid crystal composition that especially satisfies a low minimum temperature and a large negative dielectric anisotropy. A further aim is to provide a liquid crystal display device that contains such a composition. An additional aim is to provide a composition that has characteristics such as a suitable optical anisotropy which means a large optical anisotropy or a small optical anisotropy, a large negative dielectric anisotropy and a high stability to ultraviolet light, and is to provide an AM device that has characteristics such as a short response time, a large voltage holding ratio, a large contrast ratio and a long service life.

### Means for solving the Subject

The invention concerns a liquid crystal composition that has negative dielectric anisotropy and includes at least one compound selected from the group of compounds represented by formula (1) as a first component and at least one compound selected from the group of compounds represented by formula (2) as a second component, and concerns a liquid crystal display device containing this composition:

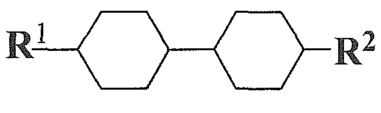
(1)

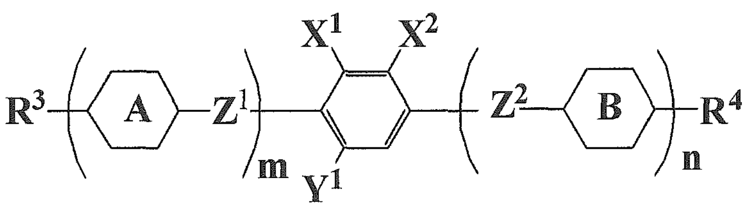
(2)

wherein $R^1$ is alkyl having 1 to 12 carbons in which one or two hydrogens have been replaced by fluorine; $R^2$, $R^3$ and $R^4$ are independently alkyl having 1 to 12 carbons, alkoxy having 1 to 12 carbons or alkenyl having 2 to 12 carbons; ring A and ring B are independently

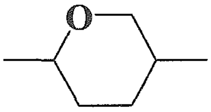 , 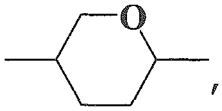 , 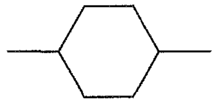 or 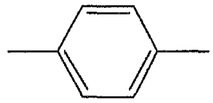 ;

$X^1$ and $X^2$ are independently fluorine or chlorine; $Y^1$ is hydrogen or methyl; $Z^1$ and $Z^2$ are independently a single bond, ethylene, methyleneoxy or carbonyloxy; and m and n are independently 0, 1, 2 or 3, and the sum of m and n is 1, 2 or 3.

**Effect of the Invention**

An advantage of the invention is a liquid crystal composition that satisfies at least one of characteristics such as a high maximum temperature of a nematic phase, a low minimum temperature of a nematic phase, a small viscosity, a suitable optical anisotropy, a large negative dielectric anisotropy, a large specific resistance, a high stability to ultraviolet light and a high stability to heat. One aspect of the invention is a liquid crystal composition that is suitably balanced between at least two of the characteristics. Another aspect is a liquid crystal display device that contains such a composition. A further aspect is a composition that has a suitable optical anisotropy, a large negative dielectric anisotropy, a high stability to ultraviolet light and so forth, and an AM device that has a short response time, a large voltage holding ratio, a large contrast ratio, a long service life and so forth.

**Best Embodiment to Carry out the Invention**

Usage of the terms in this specification is as follows. The liquid crystal composition of the invention and the liquid crystal display device of the invention may be abbreviated to "the composition" and "the device," respectively. A liquid crystal display device is a generic term for a liquid crystal display panel and a liquid crystal display module. "A liquid crystal compound" is a generic term for a compound having a liquid crystal phase such as a nematic phase or a smectic phase, and also for a compound having no liquid crystal phases but being useful as a component of a composition. This useful compound has a six-membered ring such as 1,4-cyclohexylene and 1,4-phenylene, and a rod-like molecular structure. An optically active compound and a polymerizable compound may be added to the composition. Even in the case where these compounds are liquid crystalline, the compounds are classified as an additive herein. At least one compound selected from the group of compounds represented by formula (1) may be abbreviated to "compound (1)." "Compound (1)" means one compound, or two or more compounds represented by formula (1). The same rules apply to compounds represented by other formulas. "At least one" in the case of "has been replaced" means that the position and the number of the replacement may be selected without restriction.

A higher limit of the temperature range of a nematic phase may be abbreviated to "the maximum temperature." A lower limit of the temperature range of a nematic phase may be abbreviated to "the minimum temperature." That "specific resistance is large" means that a composition has a large specific resistance at room temperature and also at a temperature close to the maximum temperature of a nematic phase in the initial stage, and that the composition has a large specific resistance at room temperature and also at a temperature close to the maximum temperature of a nematic phase even after it has been used for a long time. That "a voltage holding ratio is large" means that a device has a large voltage holding ratio at room temperature and also at a temperature close to the maximum temperature of a nematic phase in the initial stage, and that the device has a large voltage holding ratio at room temperature and also at a temperature close to the maximum temperature of a nematic phase even after it has been used for a long time. When characteristics such as optical anisotropy are explained, values obtained according to the measuring methods that are described in Examples will be used. A first component means one compound, or two or more compounds. "The ratio of the first component" is expressed as a percentage by weight (% by weight) of the first component based on the total weight of the liquid crystal composition. The same rule applies to the ratio of a second component and so forth. The ratio of an additive mixed with the composition is expressed as a percentage by weight (% by weight) or weight parts per million (ppm) based on the total weight of the liquid crystal composition.

The symbol $R^2$ is used for a plurality of compounds in the chemical formulas of component compounds. In two arbitrary compounds among these, groups selected for $R^2$ may be the same or different. In one case, for example, $R^2$ of compound (1) is ethyl and $R^2$ of compound (1-1) is ethyl. In another case, $R^2$ of compound (1) is ethyl and $R^2$ . of compound (1-1) is propyl. The same rule applies to the symbols $R^3$, $R^4$ and so forth.

The invention includes the following items.

1. A liquid crystal composition having negative dielectric anisotropy and including at least one compound selected from the group of compounds represented by formula (1) as a first component and at least one compound selected from the group of compounds represented by formula (2) as a second component:

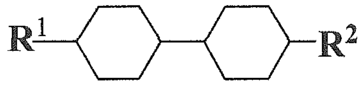

(1)

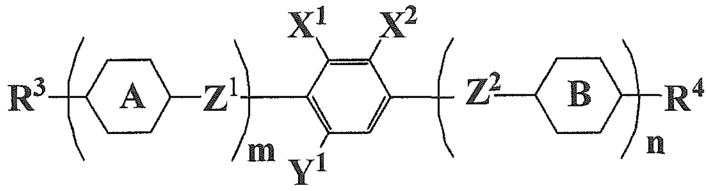

(2)

wherein $R^1$ is alkyl having 1 to 12 carbons in which one or two hydrogens have been replaced by fluorine; $R^2$, $R^3$ and $R^4$ are independently alkyl having 1 to 12 carbons, alkoxy having 1 to 12 carbons or alkenyl having 2 to 12 carbons; ring A and ring B are independently

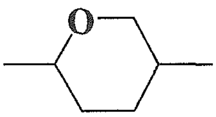

,

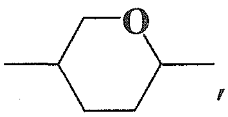

,

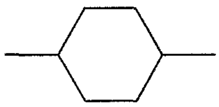

or

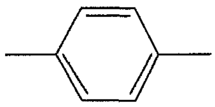

;

$X^1$ and $X^2$ are independently fluorine or chlorine; $Y^1$ is hydrogen or methyl; $Z^1$ and $Z^2$ are independently a single bond, ethylene, methyleneoxy or carbonyloxy; and m and n are independently 0, 1, 2 or 3, and the sum of m and n is 1, 2 or 3.

2. The liquid crystal composition according to item 1, wherein the first component includes at least one compound selected from the group of compounds represented by formula (1-1):

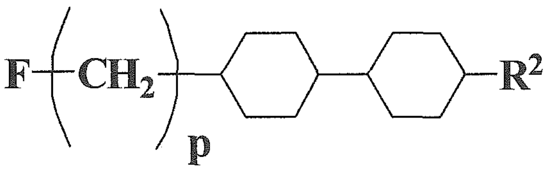

(1-1)

wherein $R^2$ is alkyl having 1 to 12 carbons, alkoxy having 1 to 12 carbons or alkenyl having 2 to 12 carbons; and p is an integer from 1 to 12.

3. The liquid crystal composition according to item 1 or 2, wherein the second component is at least one compound selected from the group of compounds represented by formula (2-1) to formula (2-12):

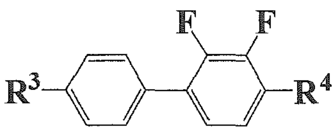

(2-1)

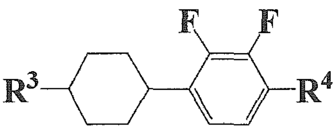

(2-2)

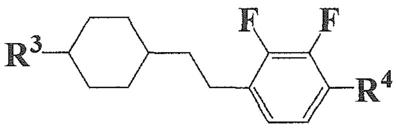

(2-3)

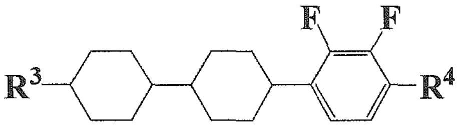

(2-4)

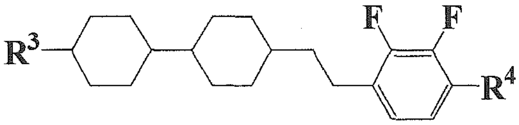

(2-5)

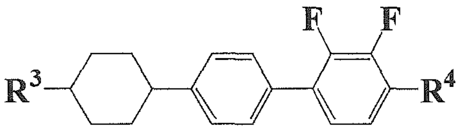

(2-6)

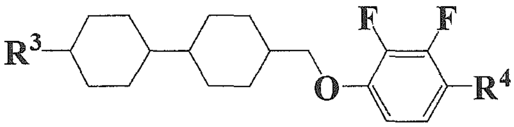

(2-7)

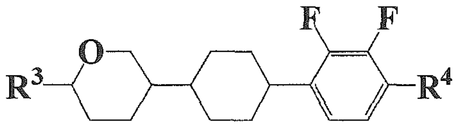

(2-8)

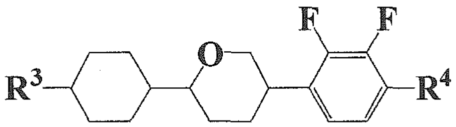

(2-9)

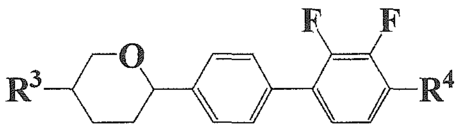

(2-10)

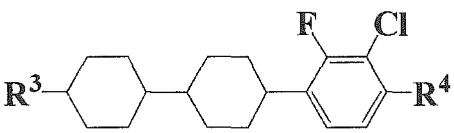

(2-11)

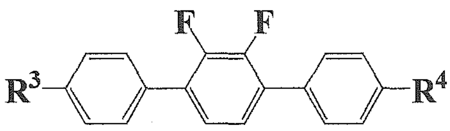

(2-12)

wherein $R^3$ and $R^4$ are independently alkyl having 1 to 12 carbons, alkoxy having 1 to 12 carbons or alkenyl having 2 to 12 carbons.

4. The liquid crystal composition according to item 2, wherein the second component is at least one compound selected from the group of compounds represented by formula (2-1) according to item 3.

5. The liquid crystal composition according to item 2, wherein the second component is at least one compound selected from the group of compounds represented by formula (2-4) according to item 3.

6. The liquid crystal composition according to item 2, wherein the second component is at least one compound selected from the group of compounds represented by formula (2-6) according to item 3.

7. The liquid crystal composition according to item 2, wherein the second component is at least one compound selected from the group of compounds represented by formula (2-7) according to item 3.

8. The liquid crystal composition according to item 2, wherein the second component is at least one compound selected from the group of compounds represented by formula (2-9) according to item 3.

9. The liquid crystal composition according to any one of items 1 to 8, wherein the ratio of the first component is in the range of 5% by weight to 80% by weight, and the ratio of the second component is in the range of 10% by weight to 95% by weight based on the total weight of the liquid crystal composition.

10. The liquid crystal composition according to any one of items 1 to 9, further including at least one compound selected from the group of compounds represented by formula (3) as a third component:

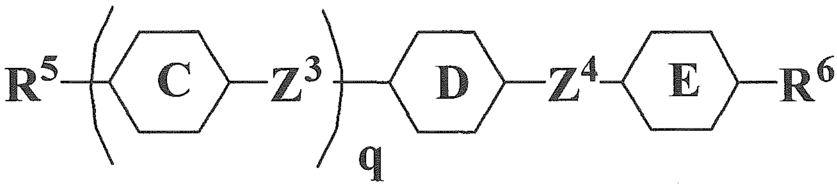

(3)

wherein $R^5$ and $R^6$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine; ring C, ring D and ring E are independently 1,4-cyclohexylene, 1,4-phenylene, 2-fluoro-1,4-phenylene or 3-fluoro-1,4-phenylene; $Z^3$ and $Z^4$ are independently a single bond, ethylene, methyleneoxy or carbonyloxy; and q is 0, 1 or 2.

11. The liquid crystal composition according to item 10, wherein the third component is at least one compound selected from the group of compounds represented by formula (3-1) to formula (3-13):

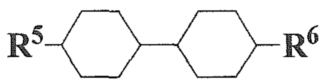

(3-1)

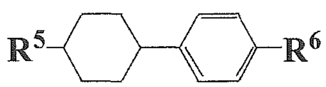

(3-2)

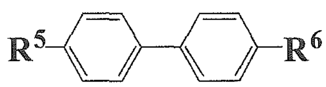

(3-3)

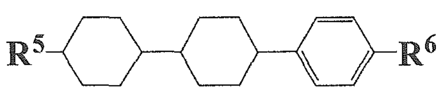

(3-4)

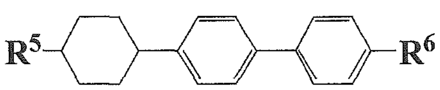

(3-5)

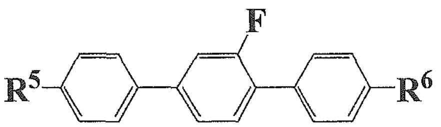

(3-6)

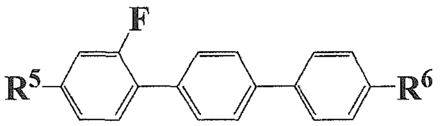

(3-7)

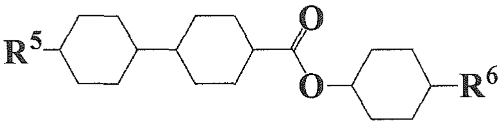

(3-8)

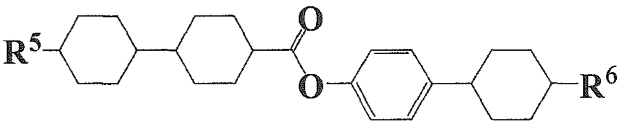

(3-9)

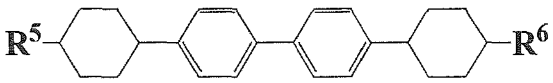

(3-10)

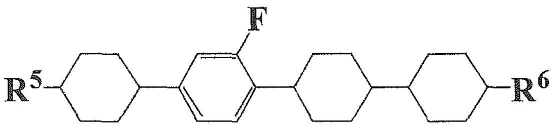

(3-11)

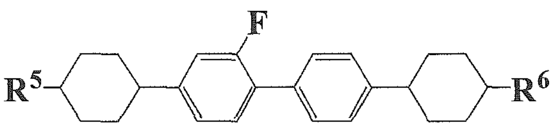

(3-12)

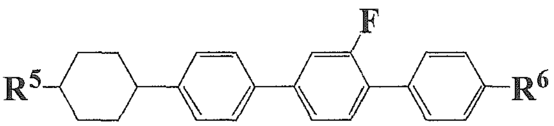

(3-13)

wherein $R^5$ and $R^6$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine.

12. The liquid crystal composition according to item 10, wherein the third component is at least one compound selected from the group of compounds represented by formula (3-1) according to item 11.

13. The liquid crystal composition according to item 10, wherein the third component is a mixture of at least one compound selected from the group of compounds represented by formula (3-1) according to item 11 and at least one compound selected from the group of compounds represented by formula (3-7) according to item 11.

14. The liquid crystal composition according to item 10, wherein the third component is a mixture of at least one compound selected from the group of compounds represented by formula (3-3) according to item 11 and at least one compound selected from the group of compounds represented by formula (3-7) according to item 11.

15. The liquid crystal composition according to item 10, wherein the third component is a mixture of at least one compound selected from the group of compounds represented by formula (3-1) according to item 11, at least one compound selected from the group of compounds represented by formula (3-4) according to item 11 and at least one compound selected from the group of compounds represented by formula (3-13) according to item 11.

16. The liquid crystal composition according to any one of items 10 to 15, wherein the ratio of the third component is in the range of 10% by weight to 80% by weight based on the total weight of the liquid crystal composition.
17. The liquid crystal composition according to any one of items 1 to 16, further including at least one compound selected from the group of compounds represented by formula (4) as a fourth component:

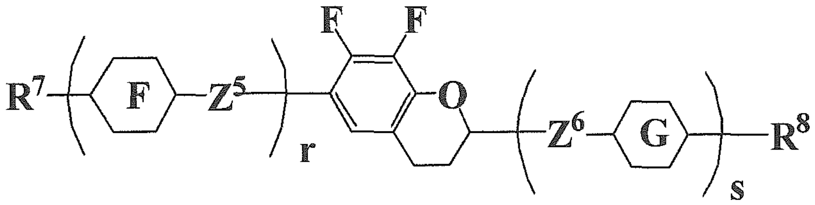
(4)

wherein $R^7$ and $R^8$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine; ring F and ring G are independently 1,4-cyclohexylene or 1, 4-phenylene; $Z^5$ and $Z^6$ are independently a single bond, ethylene, methyleneoxy or carbonyloxy; and r and s are independently 0, 1, 2 or 3, and the sum of r and s is 1, 2 or 3.
18. The liquid crystal composition according to item 17, wherein the fourth component is at least one compound selected from the group of compounds represented by formula (4-1) to formula (4-5):

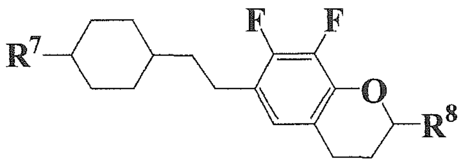
(4-1)

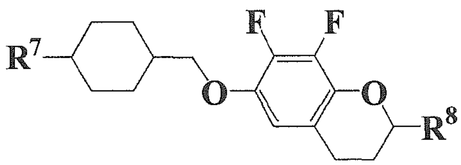
(4-2)

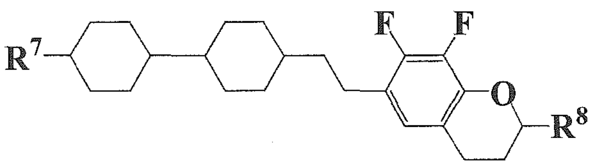
(4-3)

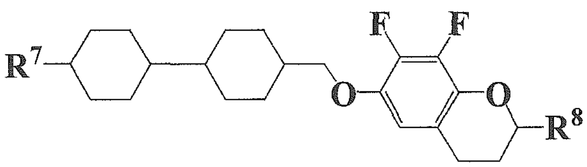
(4-4)

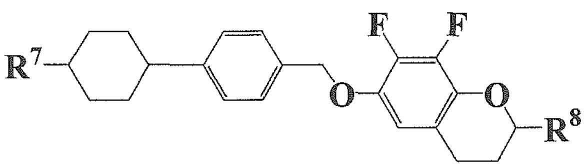
(4-5)

wherein $R^7$ and $R^8$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine.

19. The liquid crystal composition according to item 17, wherein the fourth component is at least one compound selected from the group of compounds represented by formula (4-4) according to item 18.
20. The liquid crystal composition according to any one of items 17 to 19, wherein the ratio of the fourth component is in the range of 5% by weight to 40% by weight based on the total weight of the liquid crystal composition.
21. The liquid crystal composition according to any one of items 1 to 20, wherein the maximum temperature of a nematic phase is 70 °C or higher, the optical anisotropy (25 °C) at a wavelength of 589 nanometers is 0.08 or more, and the dielectric anisotropy (25 °C) at a frequency of 1 kHz is -2 or less.
22. A compound represented by formula (1-1-1):

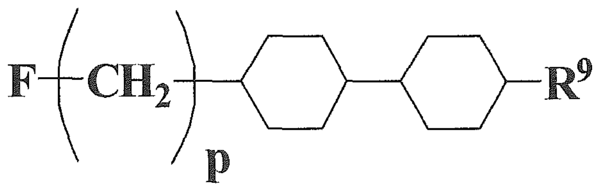

(1-1-1)

wherein p is an integer from 1 to 12; and $R^9$ is alkenyl having 2 to 12 carbons.
23. A liquid crystal display device containing the liquid crystal composition according to any one of items 1 to 21.
24. The liquid crystal display device according to item 23, wherein an operating mode of the liquid crystal display device is a VA mode, an IPS mode, an FFS mode or a PSA mode, and a driving mode of the liquid crystal display device is an active matrix mode.
25. Use of the liquid crystal composition according to any one of items 1 to 21 for a liquid crystal display device.
26. The compound represented by formula (1-1-1) according to item 22, wherein p is an integer from 1 to 12; and $R^9$ is alkenyl having 2 to 3 carbons.

The invention further includes the following items: (1) the composition described above, further including an optically active compound; (2) the composition described above, further including an additive, such as an antioxidant, an ultraviolet light absorber and an antifoaming agent; (3) an AM device containing the composition described above; (4) a device containing the composition described above and having a mode of TN, ECB, OCB, IPS, FFS, VA or PSA; (5) a transmission-type device containing the composition described above; (6) use of the composition described above, as a composition having a nematic phase; and (7) use of the composition prepared by the addition of an optically active compound to the composition described above, as an optically active composition.
The composition of the invention will be explained in the following order. First, the constitution of component compounds in the composition will be explained. Second, the main characteristics of the component compounds and the main effects of these compounds on the composition will be explained. Third, a combination of the components in the composition, a desirable ratio of the components and its basis will be explained. Fourth, a desirable embodiment of the component compounds will be explained. Fifth, specific examples of the component compounds will be shown. Sixth, additives that may be mixed with the composition will be explained. Seventh, methods for synthesizing the component compounds will be explained. Last, the use of the composition will be explained.
First, the constitution of component compounds in the composition will be explained. The compositions of the invention are classified into composition A and composition B. Composition A may further include any other liquid crystal compound, an additive and an impurity, in addition to liquid crystal compounds selected from compound (1), compound (2), compound (3) and compound (4). "Any other liquid crystal compound" is a liquid crystal compound that is different from compound (1), compound (2), compound (3) and compound (4). Such a compound is mixed with the composition for the purpose of further adjusting the characteristics. Of any other liquid crystal compound, a smaller amount of a cyano compound is desirable in view of its stability to heat or ultraviolet light. A more desirable ratio of the cyano compound is 0% by weight. The additive includes an optically active compound, an antioxidant, an ultraviolet light absorber, a coloring matter, an antifoaming agent, a polymerizable compound and a polymerization initiator. The impurity is compounds and so forth which have contaminated component compounds in a process such as their synthesis. Even in the case where the compound is liquid crystalline, it is classified as an impurity herein.
Composition B consists essentially of compounds selected from the group of compound (1), compound (2), compound (3) and compound (4). The term "essentially" means that the composition may include an additive and an impurity, but does not include any liquid crystal compound that is different from those compounds. Composition B has a smaller number of components than composition A. Composition B is preferable to composition A in view of cost reduction. Composition A is preferable to composition B in view of the fact that physical properties can be further adjusted by adding any other liquid crystal compound.
Second, the main characteristics of the component compounds and the main effects of these compounds on the characteristics of the composition will be explained. The main characteristics of the component compounds are

summarized in Table 2 on the basis of the effects of the invention. In Table 2, the symbol L stands for "large" or "high", the symbol M stands for "medium", and the symbol S stands for "mall" or "low." The symbols L, M and S are classifications based on a qualitative comparison among the component compounds, and 0 (zero) means that "a value is nearly zero."

**Table 2. Characteristics of Compounds**

| Compounds | Compound (1) | Compound (2) | Compound (3) | Compound (4) |
|---|---|---|---|---|
| Maximum Temperature | M | M-L | S-L | M-L |
| Viscosity | S | M-L | S-M | M-L |
| Optical Anisotropy | S | M-L | S-L | M-L |
| Dielectric Anisotropy | S | M-L | 0 | L |
| Specific Resistance | L | L | L | L |

The main effects of the component compounds on the characteristics of the composition upon mixing the component compounds with the composition are as follows. Compound (1) increases the absolute value of the dielectric anisotropy and decreases the minimum temperature. Compound (2) increases the absolute value of the dielectric anisotropy. Compound (3) increases the maximum temperature, decreases the minimum temperature and decreases the viscosity. Compound (4) increases the absolute value of the dielectric anisotropy and decreases the minimum temperature.

Third, a combination of the components in the composition, a desirable ratio of the components and its basis will be explained. A combination of the components in the composition is the first and second components, the first, second and third components, the first, second and fourth components, and the first, second, third and fourth components. A desirable combination of the components in the composition is the first, second and third components.

A desirable ratio of the first component is 5% by weight or more for increasing the absolute value of the dielectric anisotropy and for decreasing the minimum temperature, and 80% by weight or less for decreasing the minimum temperature. A more desirable ratio is in the range of 10% by weight to 60% by weight. An especially desirable ratio is in the range of 20% by weight to 40% by weight.

A desirable ratio of the second component is 10% by weight or more for increasing the absolute value of the dielectric anisotropy and 95% by weight or less for decreasing the viscosity. A more desirable ratio is in the range of 20% by weight to 80% by weight. An especially desirable ratio is in the range of 30% by weight to 60% by weight.

A desirable ratio of the third component is 10% by weight or more for increasing the maximum temperature or for decreasing the viscosity, and 80% by weight or less for decreasing the minimum temperature. A more desirable ratio is in the range of 20% by weight to 60% by weight. An especially desirable ratio is in the range of 30% by weight to 50% by weight.

A desirable ratio of the fourth component is 5% by weight or more for increasing the absolute value of the dielectric anisotropy and 40% by weight or less for decreasing the viscosity. A more desirable ratio is in the range of 10% by weight to 35% by weight. An especially desirable ratio is in the range of 15% by weight to 30% by weight.

Fourth, a desirable embodiment of the component compounds will be explained.

$R^1$ is alkyl having 1 to 12 carbons in which one or two hydrogen atoms have been replaced by a fluorine atom; $R^2$, $R^3$ and $R^4$ are independently alkyl having 1 to 12 carbons, alkoxy having 1 to 12 carbons or alkenyl having 2 to 12 carbons; $R^5$, $R^6$, $R^7$ and $R^8$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine; and $R^9$ is alkenyl having 2 to 12 carbons. Desirable $R^1$ is alkyl having 1 to 12 carbons in which terminal one or two hydrogens have been replaced by fluorine for increasing the absolute value of the dielectric anisotropy, and alkyl having 1 to 3 carbons in which a hydrogen atom has been replaced by a fluorine atom for decreasing the viscosity. Desirable $R^2$, $R^3$, $R^5$, $R^6$ and $R^7$ are alkyl having 1 to 12 carbons for increasing the stability to ultraviolet light or heat or alkenyl having 2 to 12 carbons for decreasing the viscosity. Desirable $R^4$ and $R^8$ are alkyl having 1 to 12 carbons for increasing the stability to ultraviolet light or heat or alkoxy having 1 to 12 carbons for increasing the absolute value of the dielectric anisotropy. Desirable $R^9$ is alkenyl having 2 to 5 carbons for decreasing the viscosity.

Desirable alkyl is methyl, ethyl, propyl, butyl, pentyl, hexyl, heptyl or octyl. More desirable alkyl is ethyl, propyl, butyl, pentyl or heptyl for decreasing the viscosity.

Desirable alkoxy is methoxy, ethoxy, propoxy, butoxy, pentyloxy, hexyloxy or heptyloxy. More desirable alkoxy is methoxy or ethoxy for decreasing the viscosity.

Desirable alkenyl is vinyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl or 5-hexenyl. More desirable alkenyl is vinyl, 1-propenyl,

3-butenyl or 3-pentenyl for decreasing the viscosity. A desirable configuration of -CH=CHin the alkenyl depends on the position of the double bond. Trans is preferable in the alkenyl such as 1-propenyl, 1-butenyl, 1-pentenyl, 1-hexenyl, 3-pentenyl and 3-hexenyl for decreasing the viscosity for instance. Cis is preferable in the alkenyl such as 2-butenyl, 2-pentenyl and 2-hexenyl. In the alkenyl, straight-chain alkenyl is preferable to branched-chain alkenyl.

Desirable examples of alkenyl in which at least one hydrogen has been replaced by fluorine are 2,2-difluorovinyl, 3,3-difluoro-2-propenyl, 4,4-difluoro-3-butenyl, 5,5-difluoro-4-pentenyland6,6-difluoro-5-hexenyl. More desirable examples are 2,2-difluorovinyl and 4,4-difluoro-3-butenyl for decreasing the viscosity.

m and n are independently 0, 1, 2 or 3, and the sum of m and n is 1, 2 or 3. Desirable m and n are 1 for decreasing the minimum temperature and are 2 for increasing the maximum temperature.

p is an integer from 1 to 12. Desirable p is an integer from 1 to 3 for decreasing the viscosity. q is 0, 1 or 2. Desirable q is 1 for decreasing the viscosity and is 2 for increasing the maximum temperature.

r and s are independently 0, 1, 2 or 3, and the sum of r and s is 1, 2 or 3. Desirable r is 2 for increasing the maximum temperature. Desirable s is 0 for decreasing the minimum temperature.

Ring A and ring B are independently

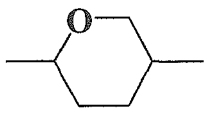 , 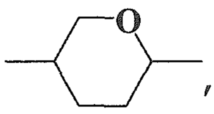 , 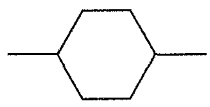 or 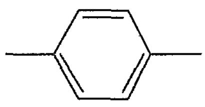 ;

arbitrary two ring A may be the same or different when m is 2 or 3, and arbitrary two ring B may be the same or different when n is 2 or 3. Desirable ring A or ring B is 1, 4-cyclohexylene for increasing the maximum temperature, 1,4-phenylene for increasing the optical anisotropy, and

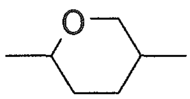 or 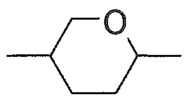

for increasing the absolute value of the dielectric anisotropy, and is preferably

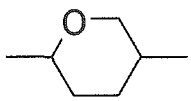 .

Ring C, ring D and ring E are independently 1,4-cyclohexylene, 1,4-phenylene, 2-fluoro-1,4-phenylene or 3-fluoro-1,4-phenylene, and two ring C may be the same or different when q is 2. Desirable ring C, ring D or ring E is 1,4-cyclohexylene for decreasing the viscosity and 1,4-phenylene for increasing the optical anisotropy. Ring F and ring G are independently 1, 4-cyclohexylene or 1, 4-phenylene, arbitrary two ring F may be the same or different when r is 2 or 3, and arbitrary two ring G may be the same or different when s is 2 or 3. With regard to the configuration of 1,4-cyclohexylene, trans is preferable to cis for increasing the maximum temperature.

$X^1$ and $X^2$ are independently fluorine or chlorine. Desirable $X^1$ or $X^2$ is fluorine for decreasing the viscosity.

$Y^1$ is hydrogen or methyl. Desirable $Y^1$ is hydrogen for decreasing the viscosity.

$Z^1$ and $Z^2$ are independently a single bond, ethylene, methyleneoxy or carbonyloxy; arbitrary two $Z^1$ may be the same or different when m is 2 or 3, and arbitrary two $Z^2$ may be the same or different when n is 2 or 3. Desirable $Z^1$ and $Z^2$ are a single bond for decreasing the viscosity and methyleneoxy for increasing the absolute value of the dielectric anisotropy. $Z^3$ and $Z^4$ are independently a single bond, ethylene, methyleneoxy or carbonyloxy, and two $Z^3$ may be the same or different when q is 2. Desirable $Z^3$ is a single bond for decreasing the viscosity and carbonyloxy for increasing the maximum temperature. $Z^5$ and $Z^6$ are independently a single bond, ethylene, methyleneoxy or carbonyloxy; arbitrary two $Z^5$ may be the same or different when r is 2 or 3, and arbitrary two $Z^6$ may be the same or different when s is 2 or 3. Desirable $Z^5$ and $Z^6$ are a single bond for decreasing the viscosity and carbonyloxy for increasing the absolute value of the dielectric anisotropy.

Fifth, specific examples of the component compounds will be shown. In the desirable compounds described below, $R^9$ is alkenyl having 2 to 12 carbons. $R^{10}$ and $R^{12}$ are independently straight-chain alkyl having 1 to 12 carbons or straight-chain alkenyl having 2 to 12 carbons. $R^{11}$ is straight-chain alkyl having 1 to 12 carbons or straight-chain alkoxy having 1 to 12 carbons. $R^{13}$ is straight-chain alkyl having 1 to 12 carbons, straight-chain alkoxy having 1 to 12 carbons or straight-chain alkenyl having 2 to 12 carbons.

Desirable compound (1) is compound (1-1-1). Desirable compound (2) is compound (2-1-1) to compound (2-12-1). More desirable compound (2) is compound (2-1-1) to compound (2-10-1). Especially desirable compound (2) is compound (2-1-1) to compound (2-7-1). Desirable compound (3) is compound (3-1-1) to compound (3-13-1). More desirable compound (3) is compound (3-1-1) to compound (3-7-1), and compound (3-9-1) to compound (3-13-1). Especially desirable compound (3) is compound (3-1-1), compound (3-3-1), compound (3-4-1), compound (3-7-1), and compound (3-13-1). Desirable compound (4) is compound (4-1-1) to compound (4-5-1). More desirable compound (4) is compound (4-4-1).

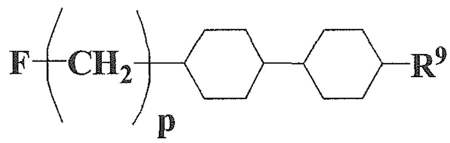

(1-1-1)

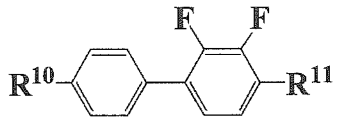

(2-1-1)

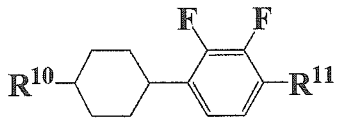

(2-2-1)

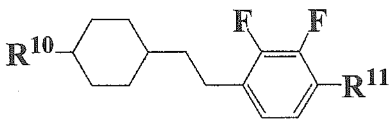

(2-3-1)

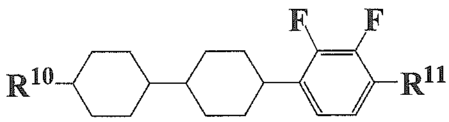

(2-4-1)

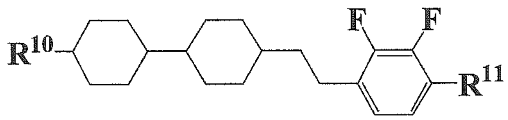

(2-5-1)

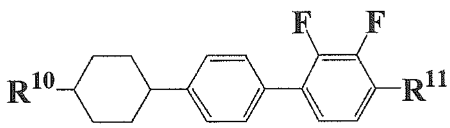

(2-6-1)

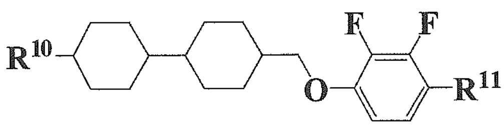

(2-7-1)

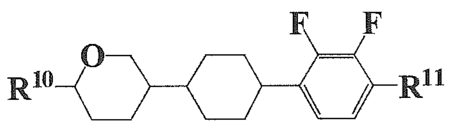

(2-8-1)

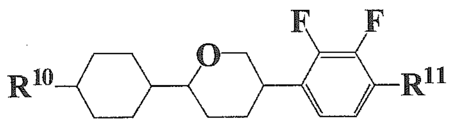

(2-9-1)

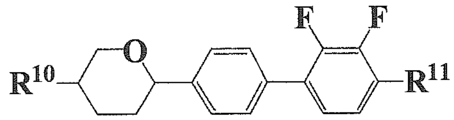

(2-10-1)

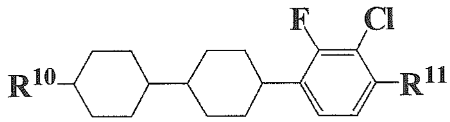

(2-11-1)

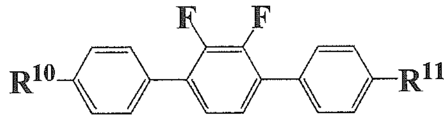

(2-12-1)

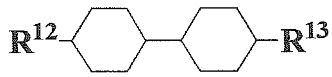

(3-1-1)

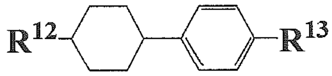

(3-2-1)

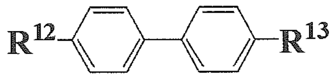

(3-3-1)

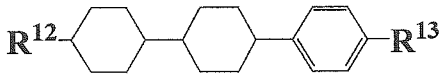

(3-4-1)

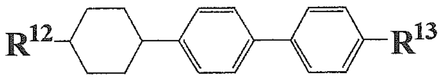

(3-5-1)

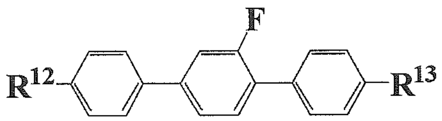

(3-6-1)

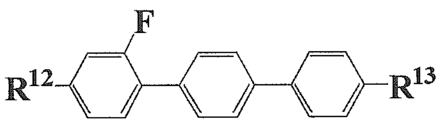

(3-7-1)

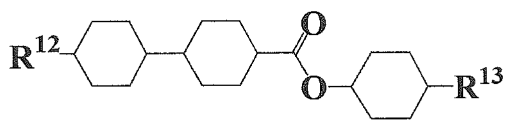

(3-8-1)

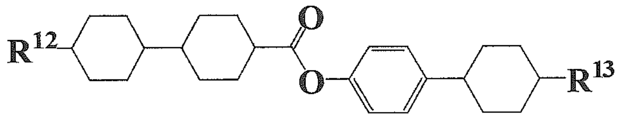

(3-9-1)

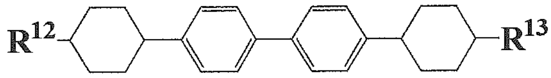

(3-10-1)

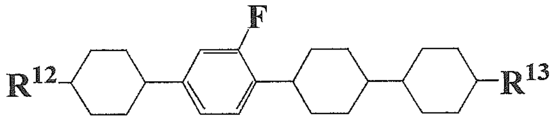

(3-11-1)

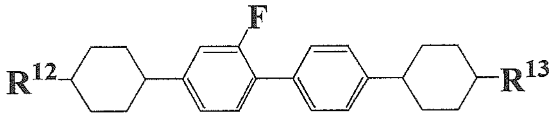

(3-12-1)

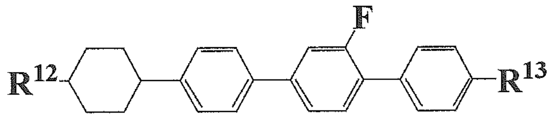

(3-13-1)

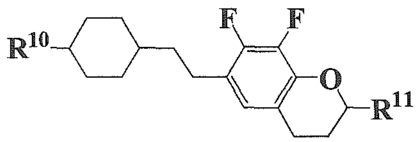

(4-1-1)

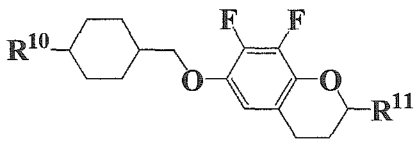

(4-2-1)

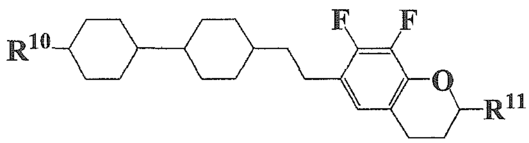

(4-3-1)

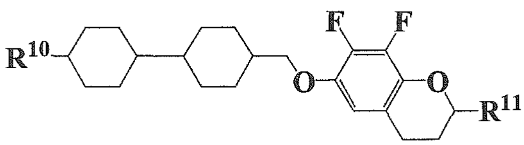

(4-4-1)

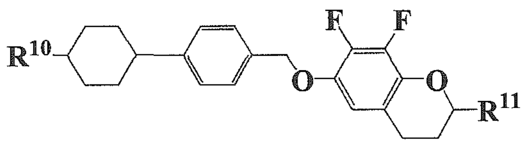

(4-5-1)

Sixth, additives that may be mixed with the composition will be explained. Such additives include an optically active compound, an antioxidant, an ultraviolet light absorber, a coloring matter, an antifoaming agent, a polymerizable compound and a polymerization initiator. The optically active compound is mixed with the composition for the purpose of inducing a helical structure and giving a twist angle in liquid crystals. Examples of such compounds include compound

(5-1) to compound (5-5). A desirable ratio of the optically active compound is 5% by weight or less, and a more desirable ratio is in the range of 0.01% by weight to 2% by weight.

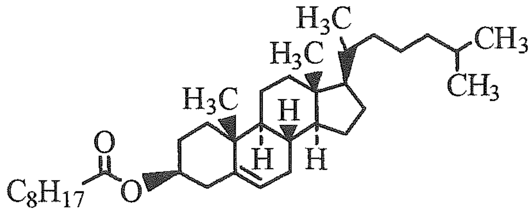

(5-1)

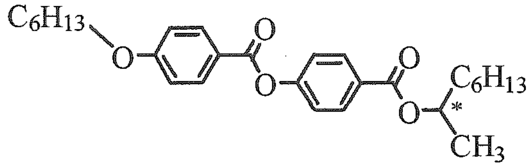

(5-2)

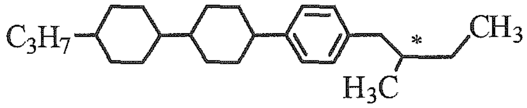

(5-3)

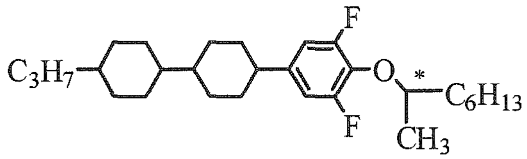

(5-4)

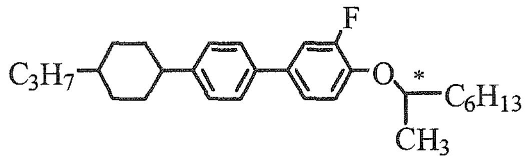

(5-5)

An antioxidant is mixed with the composition in order to prevent a decrease in specific resistance that is caused by heating under air, or to maintain a large voltage holding ratio at room temperature and also at a temperature close to the maximum temperature of a nematic phase after the device has been used for a long time.

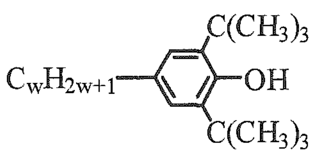

(6)

Desirable examples of the antioxidant include compound (6) where w is an integer from 1 to 9. In compound (6), desirable w is 1, 3, 5, 7 or 9. More desirable w is 1 or 7. Compound (6) where w is 1 is effective in preventing a decrease in specific resistance that is caused by heating under air, because it has a large volatility. Compound (6) where w is 7 is effective in maintaining a large voltage holding ratio at room temperature and also at a temperature close to the maximum temperature of a nematic phase even after the device has been used for a long time, because it has a small volatility. A desirable ratio of the antioxidant is approximately 50 ppm or more for achieving the effect and is approximately 600 ppm or less for avoiding a decrease in the maximum temperature or avoiding an increase in the minimum temperature. A more desirable ratio is in the range of approximately 100 ppm to approximately 300 ppm.

Desirable examples of an ultraviolet light absorber include a benzophenone derivative, a benzoate derivative and a triazole derivative. A light stabilizer such as an amine having steric hindrance is also desirable. A desirable ratio of the ultraviolet light absorber or the light stabilizer is approximately 50 ppm or more for achieving the effect and is

approximately 10,000 ppm or less for avoiding a decrease in the maximum temperature or avoiding an increase in the minimum temperature. A more desirable ratio is in the range of approximately 100 ppm to approximately 10,000 ppm.

A dichroic dye such as an azo dye or an anthraquinone dye is mixed with the composition for adjusting to a device having a guest host (GH) mode. A desirable ratio of the coloring matter is in the range of approximately 0.01% by weight to approximately 10% by weight. An antifoaming agent such as dimethyl silicone oil or methyl phenyl silicone oil is mixed with the composition for preventing foam formation. A desirable ratio of the antifoaming agent is approximately 1 ppm or more for achieving the effect and is approximately 1,000 ppm or less for avoiding a poor display. A more desirable ratio is in the range of approximately 1 ppm to approximately 500 ppm.

A polymerizable compound is mixed with the composition for adjusting to a device having a polymer sustained alignment (PSA) mode. Desirable examples of the polymerizable compound include compounds having a polymerizable group, such as acrylates, methacrylates, vinyl compounds, vinyloxy compounds, propenyl ethers, epoxy compounds (oxiranes, oxetanes) and vinyl ketones. Especially desirable examples are acrylate derivatives or methacrylate derivatives. A desirable ratio of the polymerizable compound is approximately 0.05% by weight or more for achieving the effect and is approximately 10% by weight or less for avoiding a poor display. A more desirable ratio is in the range of approximately 0.1% by weight to approximately 2% by weight. The polymerizable compound is polymerized on irradiation with ultraviolet light or the like, preferably in the presence of a suitable initiator such as a photopolymerization initiator. Suitable conditions for polymerization, a suitable type of the initiator and a suitable amount are known to a person skilled in the art and are described in the literature. For example, Irgacure 651 (registered trademark), Irgacure 184 (registered trademark) or Darocure 1173 (registered trademark) (BASF), each of which is a photoinitiator, is suitable for radical polymerization. A desirable ratio of the photopolymerization initiator is in the range of approximately 0.1% by weight to approximately 5% by weight and an especially desirable ratio is in the range of approximately 1% by weight to approximately 3% by weight based on the polymerizable compound.

Seventh, methods for synthesizing the component compounds will be explained. These compounds can be prepared by known methods. The synthetic methods will be exemplified as follows. Compound (1-1) is prepared by the method described in JP H11-035500 A (1999). Compound (2-2-1) and compound (2-3-1) are prepared by the method described in JP H02-503441 A (1990). Compound (2-9-1) is prepared by the method described in JP 2000-008040 A. Compound (3-1-1) is prepared by the method described in JP S59-070624 A (1984). Compound (4-4-1) is prepared by the method described in JP 2005-035986 A. An antioxidant is commercially available. The compound where w is 1 in formula (6) is available from Sigma-Aldrich Corporation. Compound (6) where w is 7 or the like is synthesized according to the method described in U. S. Patent No. 3,660,505.

Compounds whose synthetic methods are not described above can be prepared according to the methods described in books such as Organic Syntheses (John Wiley & Sons, Inc.), Organic Reactions (John Wiley & Sons, Inc. ), Comprehensive Organic Synthesis (Pergamon Press) and Shin Jikken Kagaku Kouza (New Experimental Chemistry Course, in English; Maruzen Co., Ltd.). The composition is prepared according to known methods using the compounds thus obtained. For example, the component compounds are mixed and dissolved in each other by heating.

Last, the use of the composition will be explained. The composition of the invention mainly has a minimum temperature of approximately -10 °C or lower, a maximum temperature of approximately 70 °C or higher, and an optical anisotropy in the range of approximately 0.07 to approximately 0.20. A device containing this composition has a large voltage holding ratio. This composition is suitable for an AM device. This composition is suitable especially for an AM device having a transmission type. The composition having an optical anisotropy in the range of approximately 0.08 to approximately 0.25, and even the composition having an optical anisotropy in the range of approximately 0.10 to approximately 0.30 may be prepared by adjusting ratios of the component compounds or by mixing with any other liquid crystal compound. This composition can be used as a composition having a nematic phase, or as an optically active composition by the addition of an optically active compound.

Desirable minimum temperature of a nematic phase in the liquid crystal composition of the invention is at least approximately 0 °C or lower, more desirable minimum temperature of a nematic phase is approximately -20 °C or lower, and especially more desirable minimum temperature of a nematic phase is approximately -30 °C or lower.

Desirable maximum temperature of a nematic phase in the liquid crystal composition of the invention is at least approximately 70 °C or higher, more desirable maximum temperature of a nematic phase is approximately 75 °C or higher, and especially more desirable maximum temperature of a nematic phase is approximately 80 °C or higher.

Desirable optical anisotropy (589 nanometers) at 25 °C in the liquid crystal composition of the invention is in the range of approximately 0.07 to approximately 0.20, more desirable optical anisotropy is in the range of approximately 0.07 to approximately 0.16, and especially more desirable optical anisotropy is in the range of approximately 0.08 to approximately 0.12.

Desirable absolute value of the dielectric anisotropy at 25 °C in the liquid crystal composition of the invention is at least approximately 1.5 or more, more desirable absolute value of the dielectric anisotropy is approximately 2 or more, and especially more desirable absolute value of the dielectric anisotropy is approximately 2.5 or more.

This composition can be used for an AM device. This composition can also be used for a PM device. The

composition can also be used for the AM device and the PM device having a mode such as PC, TN, STN, ECB, OCB, IPS, FFS, VA or PSA. It is especially desirable to use the composition for the AM device having the IPS, FFS or VA mode. These devices may be of a reflection type, a transmission type or a semi-transmission type. It is desirable to use the composition for a device having the transmission type. It can be used for an amorphous silicon-TFT device or a polycrystal silicon-TFT device. This composition is also usable for a nematic curvilinear aligned phase (NCAP) device prepared by microcapsulating the composition, and for a polymer dispersed (PD) device in which a three-dimensional network-polymer is formed in the composition.

## EXAMPLES

When a sample was a composition, the composition itself was measured, and the value obtained was described here. When the sample was a compound, a sample was prepared by mixing the compound (15% by weight) and mother liquid crystals (85% by weight). The characteristic values of the compound were calculated from values obtained by measurement, according to a method of extrapolation: (extrapolated value) = [(measured value of a sample for measurement) - 0.85 × (measured value of mother liquid crystals)]/ 0.15. When a smectic phase (or crystals) separated out in this ratio at 25 °C, the ratio of the compound to the mother liquid crystals was changed step by step in the order of (10% by weight/ 90% by weight), (5% by weight/ 95% by weight) and (1% by weight / 99% by weight). The values of the maximum temperature, the optical anisotropy, the viscosity and the dielectric anisotropy with regard to the compound were obtained by this extrapolation method.

The components of the mother liquid crystals were as follows. The ratio of each component is expressed as a percentage by weight.

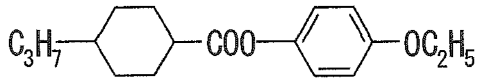
17.2%

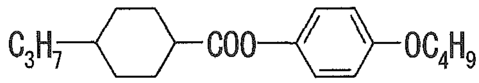
27.6%

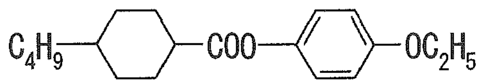
20.7%

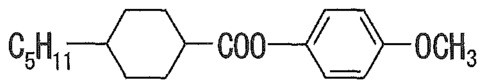
20. 7%

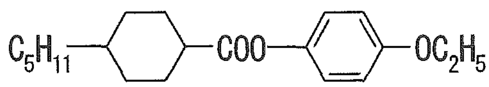
13.8%

Characteristics were measured according to the following methods. Most are methods described in the Standards of Electronic Industries Association of Japan, EIAJ·ED-2521 A or the modified method.

**Maximum Temperature of a Nematic Phase (NI; °C):** A sample was placed on a hot plate in a melting point apparatus equipped with a polarizing microscope and was heated at the rate of 1 °C per minute. The temperature was measured when part of the sample began to change from a nematic phase to an isotropic liquid. A higher limit of the temperature range of a nematic phase may be abbreviated to "the maximum temperature."

**Minimum Temperature of** a **Nematic Phase (Tc; °C) :** A sample having a nematic phase was put in glass vials and then kept in freezers at temperatures of 0 °C, -10 °C, -20 °C, -30 °C and -40 °C for 10 days, and then the liquid crystal phases were observed. For example, when the sample maintained the nematic phase at -20 °C and changed to crystals or a smectic phase at -30 °C, Tc was expressed as "□ -20 °C." A lower limit of the temperature range of a nematic phase may be abbreviated to "the minimum temperature."

**Viscosity (bulk viscosity; $\eta$; measured at 20 °C; mPa·s):** An E-type viscometer was used for measurement.

**Optical Anisotropy (refractive index anisotropy; $\Delta n$; measured at 25 °C):** Measurement was carried out by use of an Abbe refractometer in which the ocular was equipped with a polarizing plate, using light at a wavelength of 589 nanometers. The surface of the main prism was rubbed in one direction, and then a sample was dropped on the main prism. A refractive index ($n\|$) was measured when the direction of polarized light was parallel to that of the rubbing. A refractive index ($n\perp$) was measured when the direction of polarized light was perpendicular to that of the rubbing. The value of optical anisotropy was calculated from the equation: $\Delta n = n\| - n\perp$.

**Dielectric Anisotropy ($\Delta\varepsilon$; measured at 25 °C):** The value of dielectric anisotropy was calculated from the equation:

$$\Delta\varepsilon = \varepsilon\| - \varepsilon\perp .$$

Dielectric constants (ε|| and ε⊥) were measured as follows.

1) Measurement of a dielectric constant (ε||): A solution of octadecyltriethoxysilane (0.16 mL) in ethanol (20 mL) was applied to a thoroughly cleaned glass substrate. The glass substrate was rotated with a spinner, and then heated at 150 °C for one hour. A sample was poured into a VA device in which the distance between the two glass substrates (cell gap) was 4 micrometers, and then the device was sealed with an adhesive curable with ultraviolet light. Sine waves (0.5 V, 1 kHz) were applied to the device, and a dielectric constant (ε||) in the major axis direction of liquid crystal molecules was measured after 2 seconds.

2) Measurement of a dielectric constant (ε⊥): A polyimide solution was applied to a thoroughly cleaned glass substrate. The glass substrate was burned, and then the resulting alignment film was subjected to rubbing. A sample was poured into a TN device in which the distance between the two glass substrates (cell gap) was 9 micrometers and the twist angle was 80 degrees. Sine waves (0.5 V, 1 kHz) were applied to the device, and a dielectric constant (ε⊥) in the minor axis direction of liquid crystal molecules was measured after 2 seconds.

**Threshold Voltage (Vth; measured at 25 °C; V):** An LCD evaluation system Model LCD-5100 made by Otsuka Electronics Co. , Ltd. was used for measurement. The light source was a halogen lamp. A sample was poured into a VA device having a normally black mode, in which the distance between the two glass substrates (cell gap) was 4 micrometers and the rubbing direction was antiparallel, and then the device was sealed with an adhesive curable with ultraviolet light. Voltages applied to the device (60 Hz, rectangular waves) were increased stepwise from 0 V to 20 V in 0.02 V increments. The device was simultaneously irradiated with light in the perpendicular direction, and the amount of light passing through the device was measured. A voltage-transmittance curve was prepared, in which the maximum amount of light corresponded to 100% transmittance and the minimum amount of light corresponded to 0% transmittance. The threshold voltage was voltage at 10% transmittance.

**Voltage Holding Ratio (VHR-1; 25 °C; %):** A TN device used for measurement had a polyimide-alignment film, and the distance between the two glass substrates (cell gap) was 5 micrometers. A sample was poured into the device, and then the device was sealed with an adhesive curable with ultraviolet light. A pulse voltage (60 microseconds at 5 V) was applied to the TN device and the device was charged. A decreasing voltage was measured for 16.7 milliseconds with a high-speed voltmeter, and area A between the voltage curve and the horizontal axis in a unit cycle was obtained. Area B was an area without the decrease. The voltage holding ratio was the percentage of area A to area B.

**Voltage Holding Ratio (VHR-2; at 80 °C; %)**: A TN device used for measurement had a polyimide-alignment film, and the distance between the two glass substrates (cell gap) was 5 micrometer. A sample was poured into the device, and then the device was sealed with an adhesive curable with ultraviolet light. A pulse voltage (60 microseconds at 5 V) was applied to the TN device and the device was charged. A decreasing voltage was measured for 16.7 milliseconds with a high-speed voltmeter, and area A between the voltage curve and the horizontal axis in a unit cycle was obtained. Area B was an area without the decrease. The voltage holding ratio was a percentage of area A to area B.

**Voltage Holding Ratio (VHR-3; at 25 °C; %):** The stability to ultraviolet light was evaluated by measuring a voltage holding ratio after irradiation with ultraviolet light. A TN device used for measurement had a polyimide-alignment film and the cell gap was 5 micrometers. A sample was poured into this device, and then the device was irradiated with light for 20 minutes. The light source was an ultra high-pressure mercury lamp USH-500D (produced by Ushio, Inc.), and the distance between the device and the light source was 20 centimeters. In the measurement of VHR-3, a decreasing voltage was measured for 16.7 milliseconds. A composition having a large VHR-3 has a high stability to ultraviolet light. The value of VHR-3 is preferably 90% or more, and more preferably 95% or more.

**Voltage Holding Ratio (VHR-4; measured at 25 °C; %):** A TN device into which a sample was poured was heated in a constant-temperature bath at 80 °C for 500 hours, and then the stability to heat was evaluated by measuring the voltage holding ratio. In the measurement of VHR-4, a decreasing voltage was measured for 16.7 milliseconds. A composition having a large VHR-4 has a high stability to heat.

**Response Time (τ; measured at 25 °C; millisecond):** An LCD evaluation system Model LCD-5100 made by Otsuka Electronics Co. , Ltd. was used for measurement. The light source was a halogen lamp. The low-pass filter was set at 5 kHz. A sample was poured into a PVA device having a normally black mode, in which the cell gap between the two glass substrates was 3.2 micrometers, and the rubbing direction was antiparallel, and then the device was sealed with an adhesive curable with ultraviolet light. A voltage that was somewhat higher than the threshold voltage was applied to the device for approximately 1 minute, and then the device was irradiated with an ultraviolet light of 23.5 $mW/cm^2$ for approximately 8 minutes while a voltage of 5. 6 V was applied. Rectangular waves (60 Hz, 10 V, 0.5 second) were

applied to the device. The device was simultaneously irradiated with light in the perpendicular direction, and the amount of light passing through the device was measured. The maximum amount of light corresponded to 100% transmittance, and the minimum amount of light corresponded to 0% transmittance. The response time was the period of time required for the change from 90% to 10% transmittance (fall time; millisecond).

**Specific Resistance (ρ; measured at 25 °C; Ω cm)**: A sample (1.0 mL) was poured into a vessel equipped with electrodes. A DC voltage (10 V) was applied to the vessel, and the DC current was measured after 10 seconds. The specific resistance was calculated from the following equation:

$$(\text{specific resistance}) = [(\text{voltage}) \times (\text{electric capacity of vessel})] / [(\text{DC current}) \times (\text{dielectric constant in vacuum})].$$

**$^1$H-NMR Analysis:** A model DRX-500 apparatus (made by Bruker BioSpin Corporation) was used for measurement. A sample produced in Example and so forth was dissolved in a deuterated solvent such as $CDCl_3$ in which the sample was soluble, and the measurement was carried out under the conditions of room temperature, 500 MHz and the accumulation of 24 scans. In the explanation of the nuclear magnetic resonance spectra, the symbols s, d, t, q and m stand for a singlet, a doublet, a triplet, a quartet and a multiplet, respectively. Tetramethylsilane (TMS) was used as a standard substance for the zero point of chemical shifts δ. $^{19}$F-NMR analysis was carried out with the same apparatus.

**Gas Chromatographic Analysis:** A gas chromatograph Model GC-14B made by Shimadzu Corporation was used for measurement. The carrier gas was helium (2 milliliters per minute). The sample injector and the detector (FID) were set to 280 °C and 300 °C, respectively. A capillary column DB-1 (length 30 meters, bore 0.32 millimeter, film thickness 0.25 micrometer, dimethylpolysiloxane as the stationary phase, non-polar) made by Agilent Technologies, Inc. was used for the separation of component compounds. After the column had been kept at 200 °C for 2 minutes, it was further heated to 280 °C at the rate of 5 °C per minute. A sample was dissolved in acetone (0.1% by weight), and 1 microliter of the solution was injected into the sample injector. A recorder was a Model C-R5A Chromatopac Integrator made by Shimadzu Corporation or its equivalent. The resulting gas chromatogram showed the retention time of peaks and the peak areas corresponding to the component compounds.

Solvents for diluting the sample may also be chloroform, hexane and so forth. The following capillary columns may also be used in order to separate the component compounds: HP-1 made by Agilent Technologies Inc. (length 30 meters, bore 0.32 millimeter, film thickness 0.25 micrometer), Rtx-1 made by Restek Corporation (length 30 meters, bore 0.32 millimeter, film thickness 0.25 micrometer), and BP-1 made by SGE International Pty. Ltd. (length 30 meters, bore 0.32 millimeter, film thickness 0.25 micrometer). A capillary column CBP1-M50-025 (length 50 meters, bore 0.25 millimeter, film thickness 0.25 micrometer) made by Shimadzu Corporation may also be used for the purpose of avoiding an overlap of peaks of the compounds.

The ratio of the liquid crystal compounds included in the composition may be calculated according to the following method. The liquid crystal compounds can be detected by means of a gas chromatograph. The ratio of peak areas in the gas chromatogram corresponds to the ratio (molar ratio) of the liquid crystal compounds. When the capillary columns described above are used, the correction coefficient of respective liquid crystal compounds may be regarded as 1 (one). Accordingly, the ratio (percentage by weight) of the liquid crystal compounds can be calculated from the ratio of peak areas.

The invention will be explained in detail by way of Examples. The invention is not limited by Examples described below. The compounds described in Comparative Examples and Examples were expressed in terms of symbols according to the definition in the following Table 3. In Table 3, the configuration of 1,4-cyclohexylene is trans. A parenthesized number next to the symbol corresponds to the number of the compound. The symbol (-) means any other liquid crystal compound. Ratios (percentage) of liquid crystal compounds mean the percentages by weight (% by weight) based on the total weight of the liquid crystal composition. The liquid crystal composition further includes an impurity. Last, the values of characteristics of the composition were summarized.

**Table 3. Method of Description of Compounds using Symbols**

| $R-(A_1)-Z_1-\cdots\cdots-Z_n-(A_n)-R'$ | | | |
|---|---|---|---|
| 1) Left-terminal Group R- | Symbol | 4) Ring Structure $-A_n-$ | Symbol |
| $C_nH_{2n+1}-$ | n- | 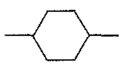 | H |
| $C_nH_{2n+1}O-$ | nO- | | |
| $C_mH_{2m+1}OC_nH_{2n}-$ | mOn- | 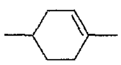 | Ch |
| $CH_2=CH-$ | V- | | |

(continued)

| R-(A$_1$)-Z$_1$-······-Z$_n$-(A$_n$)-R' | | | |
|---|---|---|---|
| 1) Left-terminal Group R- | Symbol | 4) Ring Structure -A$_n$- | Symbol |
| $C_nH_{2n+1}$-CH=CH- | nV- | 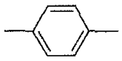 | B |
| $CH_2$=CH-$C_nH_{2n}$-<br>$C_mH_{2m+1}$-CH=CH-$C_nH_{2n}$- | Vn-<br>mVn- | 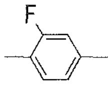 | B(2F) |
| $CF_2$=CH-<br>$CF_2$=CH-$C_nH_{2n}$- | VFF-<br>VFFn- | 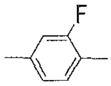 | B(F) |
| F-$C_nH_{2n}$-<br>$C_nH_{2n+1}$-$CF_2$- | Fn-<br>nCF2- | 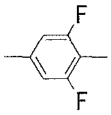 | B(F,F) |
| 2) Right-terminal Group -R' | Symbol | | |
| -$C_nH_{2n+1}$<br>-$OC_nH_{2n+1}$<br>-CH=$CH_2$ | -n<br>-On<br>-V | 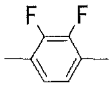 | B(2F,3F) |
| -CH=CH-$C_nH_{2n+1}$<br>-$C_nH_{2n}$-CH=$CH_2$ | -Vn<br>-nV | 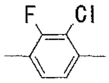 | B(2F,3CL) |
| -CH=$CF_2$ | -VFF | | |
| 3) Bonding Group -Z$_n$- | Symbol | 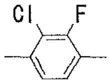 | B(2CL,3F) |
| -$C_nH_{2n}$-<br>-COO-<br>-CH=CH- | n<br>E<br>V | 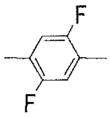 | B(3F,6F) |
| -$CH_2$O-<br>-O$CH_2$-<br>-Si$H_2$- | 1O<br>O1<br>Si | 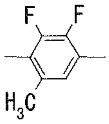 | B(2F,3F,6Me) |
| 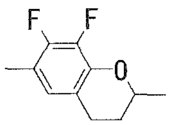 | Cro(7F,8F) | 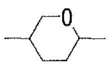 | dh |
| | | 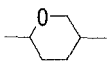 | Dh |
| 5) Examples of Description | | | |
| Example 1. F3-HH-V<br>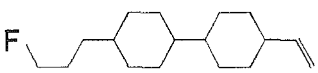 | | Example 2. 3CF2-HH-3<br>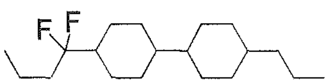 | |
| Example 3. 5-HBB(F)B-3<br>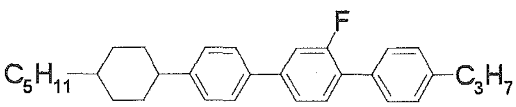 | | Example 4. 3-HBB(2F,3F)-O2<br>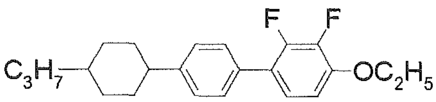 | |

**Example 1**

With regard to compound (1-1-1), the following compound was produced.

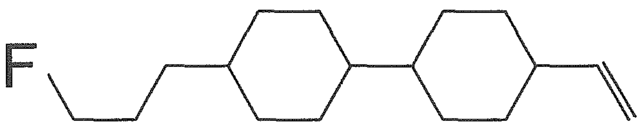

$^1$H-NMR ($CDCl_3$; δ ppm): 5.77 (ddd, 1H, J= 17 Hz, 10.5 Hz, 6.5 Hz), 4.95(ddd, 1H, J= 17 Hz, 1.5 Hz, 1.5 Hz), 4. 87 (ddd, 1H, J= 11 Hz, 1.5 Hz, 1.5 Hz), 4.42(dt, 2H, J= 47.5 Hz, 6.2 Hz), 1.92-1.82(m, 1H), 1.78-1.64(m, 10H), 1.28-1.24(m, 2H), 1.21-1.12(m, 1H), 1.10-0.84(m, 10H). $^{19}$F-NMR ($CDCl_3$ ; δ ppm): -218.0(tt, 1F, J= 95.0 Hz, 23.8 Hz).

The phase transfer temperature was Cr 3.0 SB 36.0 N 53.4 Iso, wherein Cr stands for crystals, SB stands for a smectic B phase, N stands for a nematic phase, Iso stands for isotropic liquid, and all phase transfer temperatures were shown by the unit of °C. This compound was mixed with the mother liquid crystals and the values of characteristics of the compound were obtained by the extrapolation method: NI= 33.9 °C; Δε= -1.0; Δn= 0.039.

**Example 2**

With regard to compound (1-1-1), the following compound was produced.

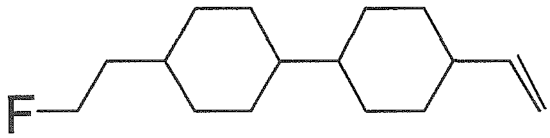

$^1$H-NMR ($CDCl_3$; δ ppm): 5.77(ddd, 1H, J= 17 Hz, 10.5 Hz, 6.5 Hz), 4.95(ddd, 1H, J= 17 Hz, 1.5 Hz, 1.5 Hz), 4.87(ddd, 1H, J= 10.5 Hz, 1.5 Hz, 1.5 Hz), 4.49(dt, 2H, J= 47.5 Hz, 6.2 Hz), 1. 92-1. 83 (m, 1H), 1. 80-1. 71 (m, 8H), 1.58 (ddt, 2H, J=26Hz, 6. 5 Hz, 6.2Hz), 1. 41-1. 33 (m, 1H), 1.10-0.89 (m, 10H). $^{19}$F-NMR ($CDCl_3$; δ ppm): -218.3(tt, 1F, J= 47.5 Hz, 52.9 Hz).

The phase transfer temperature was Cr -26.2 SB 41.4 Iso, wherein Cr stands for crystals, SB stands for a smectic phase, Iso stands for isotropic liquid, and all phase transfer temperatures were shown by the unit of °C. This compound was mixed with the mother liquid crystals and the values of characteristics of the compound were obtained by the extrapolation method: NI=15.0 °C; Δε= -0.6; Δn= 0.024.

**Example 3**

With regard to compound (1-1-1), the following compound was produced.

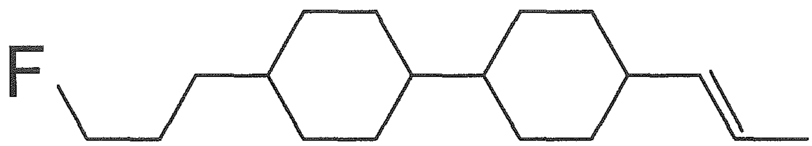

$^1$H-NMR ($CDCl_3$; δ ppm): 5.41-5.33(m, 2H), 4.42(dt, 2H, J= 47.4 Hz, J= 6.3 Hz), 1.80-1.66(m, 11H), 1.63 (d, 3H, J=4.1Hz), 1.28-1.23 (m, 2H), 1.20-1.11 (m, 1H), 1.06-0.83 (m, 10H). $^{19}$F-NMR ($CDCl_3$; δ ppm) -218.05(dt, 1F, J= 47.4 Hz, J= 24.4 Hz).

The phase transfer temperature was Cr 43.6 SB 49.8 N 87.2 Iso, wherein Cr stands for crystals, SB stands for a smectic phase, N stands for a nematic phase, Iso stands for isotropic liquid, and all phase transfer temperatures were shown by the unit of °C. This compound was mixed with the mother liquid crystals and the values of characteristics of the compound were obtained by the extrapolation method: NI= 77.0 °C; Δε= -2.6; Δn= 0.064.

**Example 4**

With regard to compound (1-1-1), the following compound was produced.

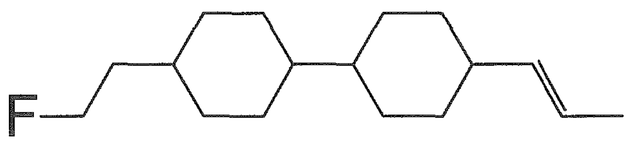

$^1$H-NMR ($CDCl_3$; δ ppm) 5. 41-5.33 (m, 2H), 4.49 (dt, 2H, J=47.4 Hz, J= 6.3 Hz), 1.80-1.71(m, 8H), 1.63(d, 3H, J= 4.9 Hz), 1.62-1.51(m, 2H), 1.41-1.25(m, 2H), 1.06-0.87(m, 10H). $^{19}$F-NMR ($CDCl_3$; δ ppm) -218.35(dt, 1F, J= 47.4 Hz, J= 24.4 Hz).

The phase transfer temperature was Cr 1.4 SB 40.4 Iso, wherein Cr stands for crystals, SB stands for a smectic phase, Iso stands for isotropic liquid, and all phase transfer temperatures were shown by the unit of °C. This compound was mixed with the mother liquid crystals and the values of characteristics of the compound were obtained by the extrapolation method: NI= 44.7 °C; Δε= -0.9; Δn= 0.049.

**Example 5**

With regard to compound (1-1-1), the following compound was produced.

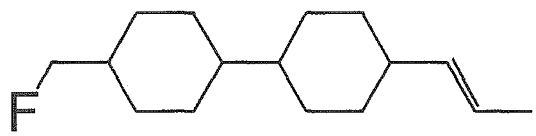

$^1$H-NMR ($CDCl_3$; δ ppm) 5.43-5.35 (m, 2H), 4.24 (dd, 2H, J=47.7 Hz, J= 6.1 Hz), 1.83-1.57(m, 11H), 1.66(d, 3H, J= 4.5 Hz), 1.10-0.97(m, 10H). $^{19}$F-NMR ($CDCl_3$; δ ppm) -222.82(dt, 1F, J=47.7 Hz, J=16.7 Hz).

The phase transfer temperature was , Cr 14.0 N 22.1 Iso, wherein Cr stands for crystals, N stands for a nematic phase, Iso stands for isotropic liquid, and all phase transfer temperatures were shown by the unit of °C. This compound was mixed with the mother liquid crystals and the values of characteristics of the compound were obtained by the extrapolation method: NI=19.7 °C; Δε=-0.8; Δn= 0.037.

**Comparative Example 1**

Composition Example 1 was selected from the compositions disclosed in JP H11-035500 A (1999). The basis of the selection was that this composition include compound (1). The components and characteristics of this composition were as follows. In Comparative Example 1, the dielectric anisotropy (Δε) is positive and thus the composition does not satisfy the subject to be solved by the invention. This applies to other Composition Examples disclosed in JP H11-035500 A (1999).

| | | |
|---|---|---|
| 3CF2-HH-3 | (1) | 10% |
| 4CF2-HH-3 | (1) | 10% |
| 7-HB(F,F)-F | (-) | 5% |
| 2-HHB(F)-F | (-) | 7% |
| 3-HHB(F)-F | (-) | 7% |
| 5-HHB(F,F)-F | (-) | 7% |
| 3-H2HB(F,F)-F | (-) | 9% |
| 5-H2HB(F,F)-F | (-) | 5% |
| 3-HH2B(F,F)-F | (-) | 8% |
| 5-HH2B(F,F)-F | (-) | 7% |
| 3-HHB(F,F)-F | (-) | 8% |
| 5-HHB(F,F)-F | (-) | 7% |
| 3-HHB-F | (-) | 5% |
| 3-HBB-F | (-) | 5% |

NI= 80.8 °C; Δn= 0.0689; η= 24.9 mPa•s; Δε= 4.7.

**Comparative Example 2**

A liquid crystal composition including a compound that is similar to compound (1) was prepared and measured by the method described above. The components and characteristics of this composition are described below. The

minimum temperature of this liquid crystal composition was in the range of -10 °C to -20 °C.

| | | |
|---|---|---|
| 3-HH-VFF | similar to (1) | 29% |
| 3-HB(2F,3F)-O2 | (2-2-1) | 10% |
| V-HB(2F,3F)-O2 | (2-2-1) | 12% |
| 3-HBB(2F,3F)-O2 | (2-6-1) | 10% |
| 5-HBB(2F,3F)-O2 | (2-6-1) | 3% |
| 3-HH1OB(2F,3F)-O2 | (2-7-1) | 11% |
| 3-HH-4 | (3-1-1) | 11% |
| 5-B(F)BB-2 | (3-7-1) | 14% |

NI= 70.8 °C; Tc<-10 °C; $\Delta n$= 0.102; $\eta$= 16.0 mPa•s; $\Delta\varepsilon$= -2.3; VHR-1= 99.1%; VHR-2= 96.3%; VHR-3= 95.5%.

**Example 6**

The compound similar to compound (1) in Comparative Example 2 was replaced by compound (1-1-1). The present composition was prepared and measured by the methods described above. The components and characteristics of this composition were as follows. Example 6 has a lower minimum temperature and a larger negative dielectric anisotropy in comparison with Comparative Example 1.

| | | |
|---|---|---|
| F3-HH-V | (1-1-1) | 29% |
| 3-HB(2F,3F)-O2 | (2-2-1) | 10% |
| V-HB(2F,3F)-O2 | (2-2-1) | 12% |
| 3-HBB(2F,3F)-O2 | (2-6-1) | 10% |
| 5-HBB (2F, 3F)-O2 | (2-6-1) | 3% |
| 3-HH1OB(2F,3F)-O2 | (2-7-1) | 11% |
| 3-HH-4 | (3-1-1) | 11% |
| 5-B (F) BB-2 | (3-7-1) | 14% |

NI= 69.8 °C; Tc< -30 °C; $\Delta n$= 0.101; $\eta$= 16.7 mPa•s; $\Delta\varepsilon$= -3.1; VHR-1= 99.2%; VHR-2= 97.8%; VHR-3= 96.6%.

**Example 7**

| | | |
|---|---|---|
| F3-HH-V | (1-1-1) | 20% |
| 3-BB(2F,3F)-O2 | (2-1-1) | 5% |
| 5-HB(2F,3F)-O2 | (2-2-1) | 5% |
| 3-H2B(2F,3F)-O2 | (2-3-1) | 11% |
| 2-HHB(2F,3F)-O2 | (2-4-1) | 3% |
| 3-HHB(2F,3F)-O2 | (2-4-1) | 5% |
| 4-HHB(2F,3F)-O2 | (2-4-1) | 5% |
| V2-HHB (2F, 3F)-O2 | (2-4-1) | 3% |
| 3-HH1OB(2F,3F)-O2 | (2-7-1) | 5% |
| 5-HH1OB(2F,3F)-O2 | (2-7-1) | 5% |

(continued)

| | | |
|---|---|---|
| 2-HH-3 | (3-1-1) | 12% |
| 3-HH-4 | (3-1-1) | 11% |
| 5-B(F)BB-2 | (3-7-1) | 10% |

NI= 71.1 °C; Tc<-30 °C; Δn= 0.087; η= 16.4 mPa•s; Δε= -3.3; VHR-1= 99.1%; VHR-2= 97.9%; VHR-3= 96.8%.

**Example 8**

| | | |
|---|---|---|
| F3-HH-V | (1-1-1) | 20% |
| 5-BB(2F,3F)-O2 | (2-1-1) | 5% |
| 5-H2B (2F, 3F)-O2 | (2-3-1) | 7% |
| 2-HHB(2F,3F)-1 | (2-4-1) | 8% |
| 3-HHB(2F,3F)-1 | (2-4-1) | 8% |
| 3-HH2B (2F, 3F)-O2 | (2-5-1) | 8% |
| 5-HBB (2F, 3F)-O2 | (2-6-1) | 3% |
| 3-DhHB(2F,3F)-O2 | (2-8-1) | 4% |
| 3-HDhB (2F, 3F)-O2 | (2-9-1) | 4% |
| 3-dhBB(2F,3F)-O2 | (2-10-1) | 3% |
| 3-HH-4 | (3-1-1) | 10% |
| 3-HH-V | (3-1-1) | 10% |
| 3-HB-O2 | (3-2-1) | 10% |

NI= 72.1 °C; Tc≤-30 °C; Δn= 0.080; η= 15.9 mPa•s; Δε= -3.2; VHR-1= 99.0%; VHR-2= 97.7%; VHR-3= 96.5%.

**Example 9**

| | | |
|---|---|---|
| F2-HH-V | (1-1-1) | 8% |
| F3-HH-V | (1-1-1) | 18% |
| F3-HH-2V1 | (1-1-1) | 10% |
| 3CF2-HH-3 | (1) | 3% |
| 3-H2B(2F,3F)-O4 | (2-3-1) | 18% |
| 2-HHB(2F,3F)-1 | (2-4-1) | 6% |
| 3-HHB(2F,3F)-1 | (2-4-1) | 7% |
| 3-HH2B(2F,3F)-O2 | (2-5-1) | 7% |
| 5-HH2B(2F,3F)-O2 | (2-5-1) | 8% |
| 3-HH1OB(2F,3F)-1 | (2-7-1) | 5% |
| 2-BB(2F,3F)B-3 | (2-12-1) | 3% |
| 2-BB(2F,3F)B-4 | (2-12-1) | 7% |

NI= 70.1 °C; Tc<-30 °C; Δn= 0.087; η= 17.1 mPa•s; Δε= -3.1; VHR-1= 99.1%; VHR-2= 97.5%; VHR-3= 96.8%.

**Example 10**

| | | |
|---|---|---|
| F3-HH-V | (1-1-1) | 20% |
| 5-HB(2F,3F)-O2 | (2-2-1) | 7% |
| 1V2-HB(2F,3F)-O2 | (2-2-1) | 3% |
| 3-H2B(2F,3F)-O4 | (2-3-1) | 12% |
| 5-HH2B (2F, 3F)-O2 | (2-5-1) | 5% |
| V2-HBB(2F,3F)-O2 | (2-6-1) | 3% |
| 3-HH1OB(2F,3F)-O2 | (2-7-1) | 5% |
| 5-HH10B(2F,3F)-O2 | (2-7-1) | 5% |
| 5-HDhB(2F,3F)-O2 | (2-9-1) | 4% |
| 3-HHB(2F,3CL)-O2 | (2-11-1) | 3% |
| 2-BB(2F,3F)B-4 | (2-12-1) | 5% |
| 3-HH-V | (3-1-1) | 5% |
| 5-HH-V | (3-1-1) | 5% |
| 3-HH-V1 | (3-1-1) | 5% |
| 1-BB-3 | (3-3-1) | 5% |
| V2-BB-1 | (3-3-1) | 3% |
| 2-BB(F)B-5 | (3-6-1) | 2% |
| 5-HBB(F)B-2 | (3-13-1) | 3% |

NI= 70.8 °C; Tc≤-30 °C; Δn= 0.098; η= 16.6 mPa•s; Δε= -3.3; VHR-1= 99.2%; VHR-2= 97.4%; VHR-3= 96.6%.

**Example 11**

| | | |
|---|---|---|
| F3-HH-V | (1-1-1) | 12% |
| 4CF2-HH-3 | (1) | 8% |
| 5-H2B(2F,3F)-O2 | (2-3-1) | 10% |
| 5-HH2B(2F,3F)-O2 | (2-5-1) | 8% |
| 3-HH1OB(2F,3F)-O2 | (2-7-1) | 4% |
| 5-HH1OB(2F,3F)-O2 | (2-7-1) | 8% |
| 2-HDhB(2F,3F)-O2 | (2-9-1) | 3% |
| 3-HH-O1 | (3-1-1) | 3% |
| 3-HH-V | (3-1-1) | 8% |
| 1-HH-2V1 | (3-1-1) | 5% |
| 3-HH-2V1 | (3-1-1) | 3% |
| 3-HB-O1 | (3-2-1) | 3% |

(continued)

| | | |
|---|---|---|
| 1-BB-5 | (3-3-1) | 7% |
| 1V2-BB-1 | (3-3-1) | 3% |
| 3-HHB-O1 | (3-4-1) | 3% |
| 3-HBB-2 | (3-5-1) | 3% |
| 3-H2Cro(7F,8F)-5 | (4-1-1) | 3% |
| 3-HH2Cro(7F,8F)-5 | (4-3-1) | 3% |
| 5-HB1OCro(7F,8F)-5 | (4-5-1) | 3% |

NI= 70.8 °C; Tc<-30 °C; Δn= 0.085; η= 19.2 mPa•s; Δε= -3.4; VHR-1= 98.9%; VHR-2= 97.1%; VHR-3= 96.2%.

**Example 12**

| | | |
|---|---|---|
| F3-HH-V | (1-1-1) | 16% |
| 3V-HB (2F, 3F)-O2 | (2-2-1) | 3% |
| 3-H2B(2F,3F)-O2 | (2-3-1) | 10% |
| 3-H2B(2F,3F)-O4 | (2-3-1) | 12% |
| 3-HH2B(2F,3F)-O2 | (2-5-1) | 8% |
| 5-HH2B(2F,3F)-O2 | (2-5-1) | 9% |
| 3-HHB(2CL,3F)-O2 | (2) | 2% |
| 3-HH-V | (3-1-1) | 12% |
| 3-HH-V1 | (3-1-1) | 3% |
| V2-BB-1 | (3-3-1) | 3% |
| V2-BB(F)B-3 | (3-6-1) | 3% |
| 5-B(F)BB-2 | (3-7-1) | 3% |
| 3-HHEH-5 | (3-8-1) | 3% |
| 3-HHEBH-3 | (3-9-1) | 3% |
| 5-H1OCroC7F,8F)-5 | (4-2-1) | 3% |
| 3-HH1OCro(7F,8F)-5 | (4-4-1) | 4% |
| 4O-Cro(7F,8F)H-3 | (4) | 3% |

NI= 71.1 °C; Tc<-30 °C; Δn= 0.086; η= 19.8 mPa•s; Δε= -3.4; VHR-1= 98.8%; VHR-2= 96.9%; VHR-3= 96.2%.

**Example 13**

| | | |
|---|---|---|
| F3-HH-V | (1-1-1) | 15% |
| 3-H2B(2F,3F)-O2 | (2-3-1) | 10% |
| 3-H2B(2F,3F)-O4 | (2-3-1) | 7% |
| 5-H2B(2F,F-O2 | (2-3-1) | 10% |
| 1V2-HHB(2F,3F)-O2 | (2-4-1) | 2% |

(continued)

| | | |
|---|---|---|
| 3-HH2B(2F,3F)-O2 | (2-5-1) | 5% |
| 3-HH1OB(2F,3F)-1 | (2-7-1) | 3% |
| 3-DhHB(2F,3F)-O2 | (2-8-1) | 3% |
| 3-HDhB(2F,3F)-O2 | (2-9-1) | 5% |
| 3-HH-V | (3-1-1) | 13% |
| 5-HB-3 | (3-2-1) | 7% |
| 1-BB-3 | (3-3-1) | 5% |
| 5-B(F)BB-3 | (3-7-1) | 3% |
| 3-HBBH-3 | (3-10-1) | 3% |
| 3-HB(F)HH-5 | (3-11-1) | 3% |
| 5-HB(F)BH-5 | (3-12-1) | 3% |
| 1O1-HBBH-5 | (-) | 3% |

NI= 70.7 °C; Tc<-30 °C; Δn= 0.090; η= 17.2 mPa•s; Δε= -3.0; VHR-1= 99.1%; VHR-2= 97.2%; VHR-3= 96.9%.

**Example 14**

| | | |
|---|---|---|
| F2-HH-V | (1-1-1) | 5% |
| F2-HH-V3 | (1-1-1) | 5% |
| F3-HH-V | (1-1-1) | 7% |
| 3-BB(2F,3F)-O2 | (2-1-1) | 7% |
| 5-HHB (2F, 3F)-O2 | (2-4-1) | 5% |
| V-HHB(2F,3F)-O2 | (2-4-1) | 5% |
| 2-HH1OB(2F,3F)-O2 | (2-7-1) | 7% |
| 3-HH1OB(2F,3F)-O2 | (2-7-1) | 5% |
| 4-HH1OB(2F,3F)-O2 | (2-7-1) | 5% |
| 2-HDhB (2F, 3F)-O2 | (2-9-1) | 3% |
| 2-BB(2F,3F)B-3 | (2-12-1) | 7% |
| 3-HH-V | (3-1-1) | 15% |
| 5-HH-V | (3-1-1) | 5% |
| 5-HB-O2 | (3-2-1) | 3% |
| 1-BB-3 | (3-3-1) | 7% |
| 3-HHB-1 | (3-4-1) | 3% |
| 5-B(F)BB-2 | (3-7-1) | 3% |
| 5-B (F)BB-3 | (3-7-1) | 3% |

NI= 72.2 °C; Tc<-30 °C; Δn= 0.102; η= 16.1 mPa•s; Δε= -3.2; VHR-1= 99.0%; VHR-2= 97.1%; VHR-3= 96.5%.

## Example 15

| | | |
|---|---|---|
| F2-HH-V1 | (1-1-1) | 3% |
| F3-HH-V1 | (1-1-1) | 5% |
| 3-BB(2F,3F)-O2 | (2-1-1) | 5% |
| 4-BB(2F,3F)-O2 | (2-1-1) | 5% |
| V-HHB(2F,3F)-O2 | (2-4-1) | 7% |
| 2-HH1OB(2F,3F)-O2 | (2-7-1) | 7% |
| 3-HH1OB(2F,3F)-O2 | (2-7-1) | 5% |
| 5-HDhB(2F,3F)-O2 | (2-9-1) | 3% |
| 3-HHB(2F,3CL)-O2 | (2-11-1) | 3% |
| 2-BB(2F,3F)B-3 | (2-12-1) | 5% |
| 5-H1OB(2F,3F)-O2 | (2) | 3% |
| 2-HH-3 | (3-1-1) | 5% |
| 3-HH-V | (3-1-1) | 17% |
| 3-HH-V1 | (3-1-1) | 5% |
| 1-BB-3 | (3-3-1) | 5% |
| 3-HHB-3 | (3-4-1) | 3% |
| V2-HHB-1 | (3-4-1) | 5% |
| 2-BB(F)B-3 | (3-6-1) | 4% |
| 2-BB(F)B-5 | (3-6-1) | 5% |

NI= 76.0 °C; Tc<-30 °C; Δn= 0.109; η= 17.7 mPa•s; Δε= -3.4; VHR-1= 99.2%; VHR-2= 97.3%; VHR-3= 96.7%.

The compositions in Example 6 to Example 15 have a lower minimum temperature and a larger negative dielectric anisotropy in comparison with those in Comparative example 1 and Comparative example 2. Therefore, the liquid crystal composition of the invention has more excellent characteristics than that shown in Patent document 1 and Patent document 2.

## Industrial Applicability

The invention provides a liquid crystal composition that satisfies at least one of characteristics such as a high maximum temperature of a nematic phase, a low minimum temperature of a nematic phase, a small viscosity, a large optical anisotropy, a large dielectric anisotropy, a large specific resistance, a high stability to ultraviolet light and a high stability to heat, or that is suitably balanced between at least two of the characteristics. A liquid crystal display device containing such a composition becomes an AM device that has a short response time, a large voltage holding ratio, a large contrast ratio, a long service life and so forth, and thus it can be used for a liquid crystal projector, a liquid crystal television and so forth.

## Claims

**1.** A liquid crystal composition having negative dielectric anisotropy and including at least one compound selected from the group of compounds represented by formula (1) as a first component and at least one compound selected from the group of compounds represented by formula (2) as a second component:

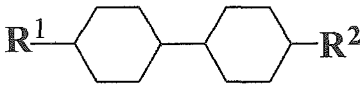

(1)

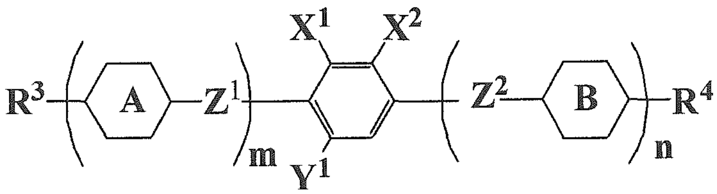

(2)

wherein $R^1$ is alkyl having 1 to 12 carbons in which one or two hydrogens have been replaced by fluorine; $R^2$, $R^3$ and $R^4$ are independently alkyl having 1 to 12 carbons, alkoxy having 1 to 12 carbons or alkenyl having 2 to 12 carbons; ring A and ring B are independently

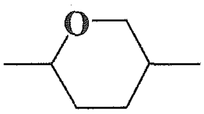 , 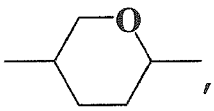 , 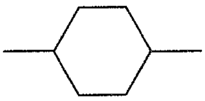 or 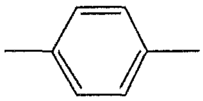 ;

$X^1$ and $X^2$ are independently fluorine or chlorine; $Y^1$ is hydrogen or methyl; $Z^1$ and $Z^2$ are independently a single bond, ethylene, methyleneoxy or carbonyloxy; and m and n are independently 0, 1, 2 or 3, and the sum of m and n is 1, 2 or 3.

2. The liquid crystal composition according to claim 1, wherein the first component includes at least one compound selected from the group of compounds represented by formula (1-1):

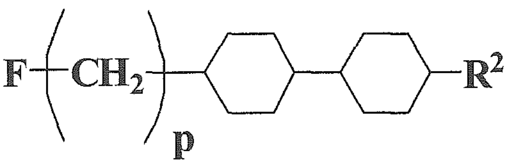

(1-1)

wherein $R^2$ is alkyl having 1 to 12 carbons, alkoxy having 1 to 12 carbons or alkenyl having 2 to 12 carbons; and p is an integer from 1 to 12.

3. The liquid crystal composition according to claim 1 or 2, wherein the second component is at least one compound selected from the group of compounds represented by formula (2-1) to formula (2-12):

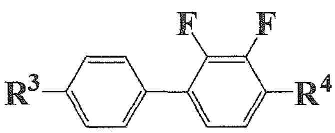

(2-1)

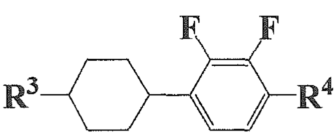

(2-2)

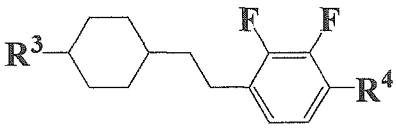

(2-3)

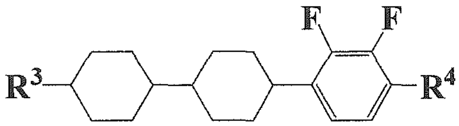

(2-4)

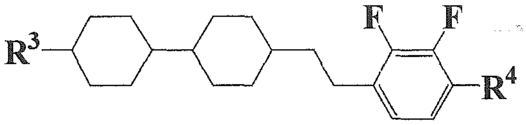

(2-5)

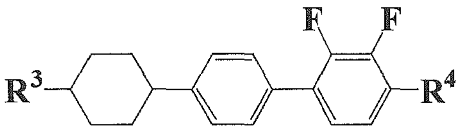

(2-6)

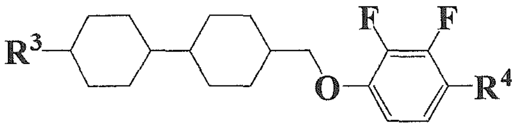

(2-7)

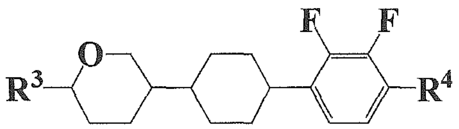

(2-8)

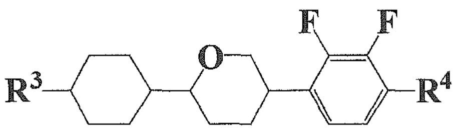

(2-9)

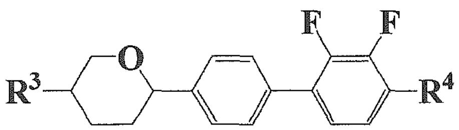

(2-10)

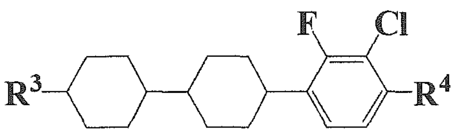

(2-11)

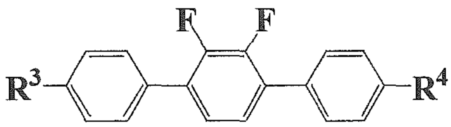

(2-12)

wherein $R^3$ and $R^4$ are independently alkyl having 1 to 12 carbons, alkoxy having 1 to 12 carbons or alkenyl having 2 to 12 carbons.

4. The liquid crystal composition according to claim 2, wherein the second component is at least one compound selected from the group of compounds represented by formula (2-1) according to claim 3.

5. The liquid crystal composition according to claim 2, wherein the second component is at least one compound selected from the group of compounds represented by formula (2-4) according to claim 3.

6. The liquid crystal composition according to claim 2, wherein the second component is at least one compound selected from the group of compounds represented by formula (2-6) according to claim 3.

7. The liquid crystal composition according to claim 2, wherein the second component is at least one compound selected from the group of compounds represented by formula (2-7) according to claim 3.

8. The liquid crystal composition according to claim 2, wherein the second component is at least one compound selected from the group of compounds represented by formula (2-9) according to claim 3.

9. The liquid crystal composition according to any one of claims 1 to 8, wherein the ratio of the first component is in the range of 5% by weight to 80% by weight, and the ratio of the second component is in the range of 10% by weight to 95% by weight based on the total weight of the liquid crystal composition.

10. The liquid crystal composition according to any one of claims 1 to 9, further including at least one compound selected from the group of compounds represented by formula (3) as a third component:

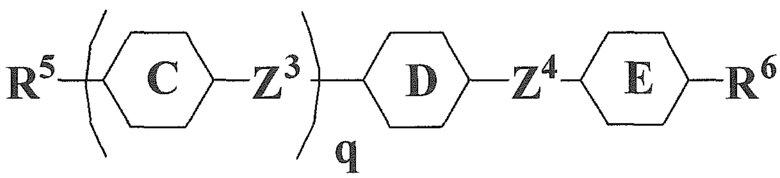

(3)

wherein $R^5$ and $R^6$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine; ring C, ring D and ring E are independently 1,4-cyclohexylene, 1,4-phenylene, 2-fluoro-1,4-phenylene or 3-fluoro-1,4-phenylene; $Z^3$ and $Z^4$ are independently a single bond, ethylene, methyleneoxy or carbonyloxy; and q is 0, 1 or 2.

11. The liquid crystal composition according to claim 10, wherein the third component is at least one compound selected from the group of compounds represented by formula (3-1) to formula (3-13):

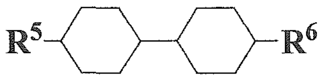

(3-1)

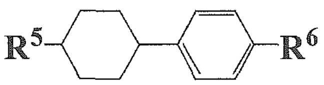

(3-2)

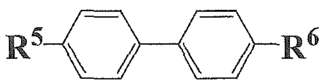

(3-3)

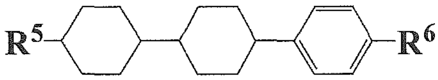
(3-4)

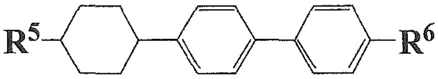
(3-5)

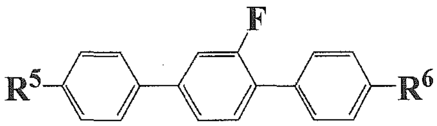
(3-6)

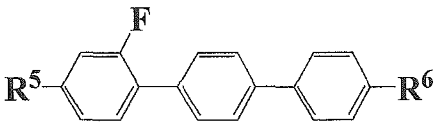
(3-7)

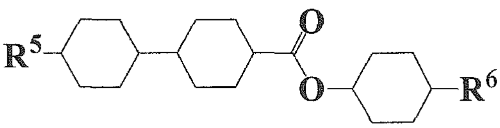
(3-8)

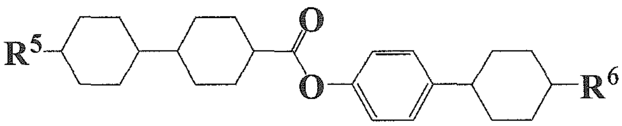
(3-9)

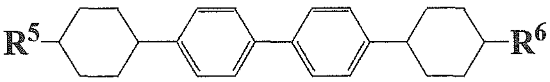
(3-10)

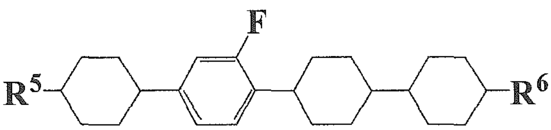
(3-11)

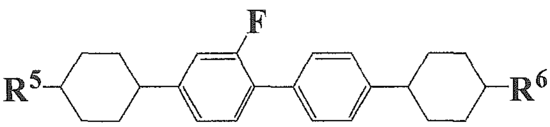
(3-12)

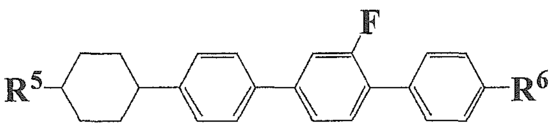
(3-13)

wherein $R^5$ and $R^6$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine.

**12.** The liquid crystal composition according to claim 10, wherein the third component is at least one compound selected

from the group of compounds represented by formula (3-1) according to claim 11.

**13.** The liquid crystal composition according to claim 10, wherein the third component is a mixture of at least one compound selected from the group of compounds represented by formula (3-1) according to claim 11 and at least one compound selected from the group of compounds represented by formula (3-7) according to claim 11.

**14.** The liquid crystal composition according to claim 10, wherein the third component is a mixture of at least one compound selected from the group of compounds represented by formula (3-3) according to claim 11 and at least one compound selected from the group of compounds represented by formula (3-7) according to claim 11.

**15.** The liquid crystal composition according to claim 10, wherein the third component is a mixture of at least one compound selected from the group of compounds represented by formula (3-1) according to claim 11, at least one compound selected from the group of compounds represented by formula (3-4) according to claim 11 and at least one compound selected from the group of compounds represented by formula (3-13) according to claim 11.

**16.** The liquid crystal composition according to any one of claims 10 to 15, wherein the ratio of the third component is in the range of 10% by weight to 80% by weight based on the total weight of the liquid crystal composition.

**17.** The liquid crystal composition according to any one of claims 1 to 16, further including at least one compound selected from the group of compounds represented by formula (4) as a fourth component:

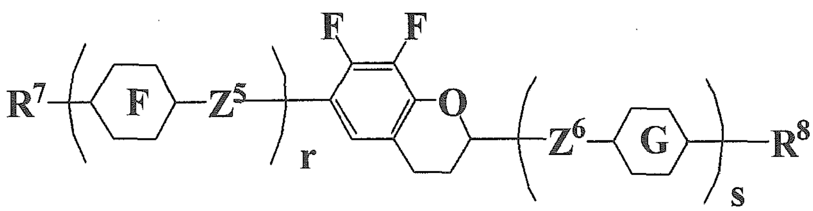

(4)

wherein $R^7$ and $R^8$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine; ring F and ring G are independently 1,4-cyclohexylene or 1, 4-phenylene; $Z^5$ and $Z^6$ are independently a single bond, ethylene, methyleneoxy or carbonyloxy; and r and s are independently 0, 1, 2 or 3, and the sum of r and s is 1, 2 or 3.

**18.** The liquid crystal composition according to claim 17, wherein the fourth component is at least one compound selected from the group of compounds represented by formula (4-1) to formula (4-5):

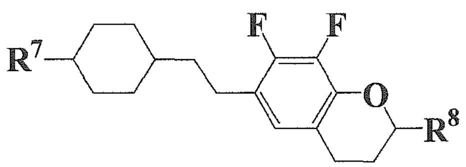

(4-1)

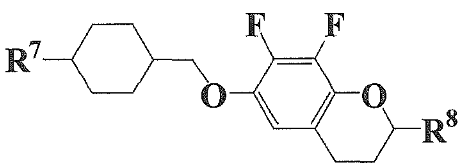

(4-2)

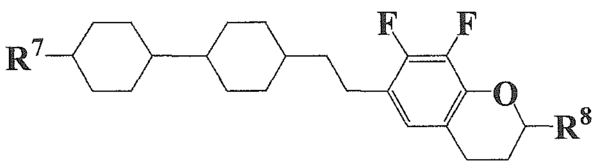

(4-3)

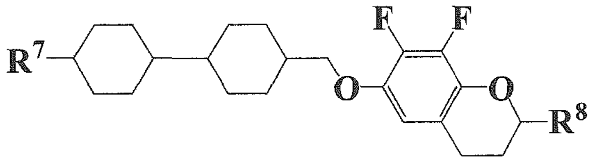

(4-4)

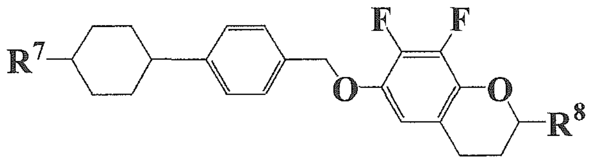

(4-5)

wherein $R^7$ and $R^8$ are independently alkyl having 1 to 12 carbons, alkoxy having 1 to 12 carbons, alkenyl having 2 to 12 carbons or alkenyl having 2 to 12 carbons in which at least one hydrogen has been replaced by fluorine.

**19.** The liquid crystal composition according to claim 17, wherein the fourth component is at least one compound selected from the group of compounds represented by formula (4-4) according to claim 18.

**20.** The liquid crystal composition according to any one of claims 17 to 19, wherein the ratio of the fourth component is in the range of 5% by weight to 40% by weight based on the total weight of the liquid crystal composition.

**21.** The liquid crystal composition according to any one of claims 1 to 20, wherein the maximum temperature of a nematic phase is 70 °C or higher, the optical anisotropy (25 °C) at a wavelength of 589 nanometers is 0.08 or more, and the dielectric anisotropy (25 °C) at a frequency of 1 kHz is -2 or less.

**22.** A compound represented by formula (1-1-1):

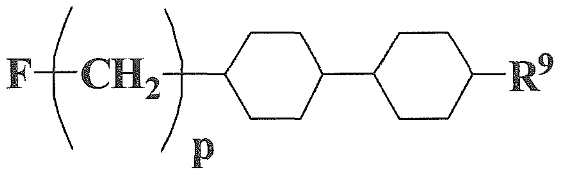

(1-1-1)

wherein p is an integer from 1 to 12; and $R^9$ is alkenyl having 2 to 12 carbons.

**23.** A liquid crystal display device containing the liquid crystal composition according to any one of claims 1 to 21.

**24.** The liquid crystal display device according to claim 23, wherein an operating mode of the liquid crystal display device is a VA mode, an IPS mode, an FFS mode or a PSA mode, and a driving mode of the liquid crystal display device is an active matrix mode.

**25.** Use of the liquid crystal composition according to any one of claims 1 to 21 for a liquid crystal display device.

**26.** The compound represented by formula (1-1-1) according to claim 22, wherein p is an integer from 1 to 12; and $R^9$ is alkenyl having 2 to 3 carbons.

## INTERNATIONAL SEARCH REPORT

International application No.
PCT/JP2012/059186

A. CLASSIFICATION OF SUBJECT MATTER
*C09K19/42*(2006.01)i, *C07C22/00*(2006.01)i, *C09K19/12*(2006.01)i, *C09K19/30*(2006.01)i, *C09K19/34*(2006.01)i, *G02F1/13*(2006.01)i

According to International Patent Classification (IPC) or to both national classification and IPC

B. FIELDS SEARCHED

Minimum documentation searched (classification system followed by classification symbols)
C09K19/42, C07C22/00, C09K19/12, C09K19/30, C09K19/34, G02F1/13

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 11-035500 A (Chisso Corp.), 09 February 1999 (09.02.1999), claims; example 8 (compound 255) & US 5779936 A & EP 742275 A1 & WO 1995/020021 A1 & DE 69430213 D & AU 8116394 A & KR 10-1997-0700754 A & TW 348182 A | 22,26<br>1-21,23-25 |
| A | WO 2010/084810 A1 (Chisso Corp.), 29 July 2010 (29.07.2010), claims; examples 5, 8, 9 & TW 201028460 A | 1-26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 May, 2012 (23.05.12) | 05 June, 2012 (05.06.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2012/059186

C (Continuation). DOCUMENTS CONSIDERED TO BE RELEVANT

| Category* | Citation of document, with indication, where appropriate, of the relevant passages | Relevant to claim No. |
|---|---|---|
| A | WO 2010/029843 A1 (Chisso Corp.), 18 March 2010 (18.03.2010), claims; examples 5, 7, 14, 17 & EP 2325280 A1 & CN 102144017 A & KR 10-2011-0053343 A & TW 201016831 A & US 20110149226 A1 | 1-26 |
| A | WO 2011/040170 A1 (Chisso Corp.), 07 April 2011 (07.04.2011), claims; examples 7, 9 & TW 201111486 A | 1-26 |
| A | WO 2008/114821 A1 (Chisso Corp.), 25 September 2008 (25.09.2008), claims; example 12 & US 2008/0303001 A1 & EP 2128225 A1 & CN 101679867 A & KR 10-2010-0014471 A & TW 200844217 A | 1-26 |
| A | JP 2010-535910 A (Merck Patent GmbH), 25 November 2010 (25.11.2010), claims; examples & US 2011/0193020 A1 & EP 2176376 A1 & WO 2009/021671 A1 & DE 102008036808 A & KR 10-2010-0066504 A & CN 101796162 A & TW 200918646 A | 1-26 |
| A | JP 5-070777 A (Merck Patent GmbH), 23 March 1993 (23.03.1993), claims; examples & US 5308538 A & EP 494368 A2 & DE 4119292 A & KR 10-0221092 B | 1-26 |
| A | JP 2010-215609 A (Chisso Corp.), 30 September 2010 (30.09.2010), examples; claims & WO 2010/095493 A1 & TW 201037062 A | 1-26 |
| A | JP 2003-201477 A (Merck Patent GmbH), 18 July 2003 (18.07.2003), claims; examples & US 2003/0186002 A1 & EP 1310474 A1 & DE 10250844 A & KR 10-2003-0038527 A & TWB 00I299357 | 1-26 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11035500 A
- JP H03505742 A
- JP H02503441 A
- JP 2000008040 A
- JP S59070624 A
- JP 2005035986 A
- US 3660505 A


### Non-patent literature cited in the description


- Organic Syntheses. John Wiley & Sons, Inc,
- Organic Reactions. John Wiley & Sons, Inc,
- Comprehensive Organic Synthesis. Pergamon Press
- Shin Jikken Kagaku Kouza. Maruzen Co., Ltd,